(19) Europäisches Patentamt
European Patent Office
Office européen des brevets

(11) Veröffentlichungsnummer: **0 056 161**
**A2**

(12) # EUROPÄISCHE PATENTANMELDUNG

(21) Anmeldenummer: **81110836.4**

(22) Anmeldetag: **30.12.81**

(51) Int. Cl.³: **C 07 C 131/105**, C 07 C 161/00, C 07 D 273/00, C 07 D 413/12, C 07 D 307/54, C 07 D 521/00, A 01 N 37/52 // (C07D521/00, 233/56, 249/08, 307/28, 309/28, 333/36, 335/02, 407/12)

(30) Priorität: **13.01.81 DE 3100728**

(43) Veröffentlichungstag der Anmeldung: **21.07.82 Patentblatt 82/29**

(84) Benannte Vertragsstaaten: **AT BE CH DE FR GB IT LI NL SE**

(71) Anmelder: **BAYER AG, Zentralbereich Patente, Marken und Lizenzen, D-5090 Leverkusen 1, Bayerwerk (DE)**

(72) Erfinder: **Stetter, Jörg, Dr., Gellertweg 4, D-5600 Wuppertal 1 (DE)**
Erfinder: **Führer, Wolfgang, Dr., Wehrstrasse 28, D-5202 Hennef 1 (DE)**
Erfinder: **Brandes, Wilhelm, Dr., Eichendorffstrasse 3, D-5653 Leichlingen 1 (DE)**

(54) **Hydroximsäureester, Verfahren zu ihrer Herstellung sowie ihre Verwendung als Fungizide.**

(57) Die vorliegende Erfindung betrifft neue Hydroximsäureester, mehrere Verfahren zu ihrer Herstellung und ihre Verwendung als Fungizide.

Die Verbindungen der allgemeinen Formel

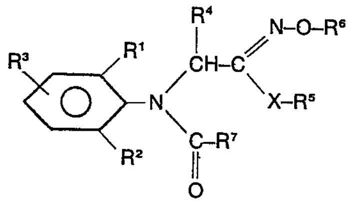

(I)

in welcher

$R^1$, $R^2$, $R^3$, $R^4$, $R^5$, $R^6$, $R^7$ und X die in der Beschreibung angegebene Bedeutung besitzen, werden erhalten, wenn man z.B. Anilin-Derivate mit Säurechloriden oder -bromiden bzw. -anhydriden in Gegenwart eines Verdünnungsmittels und gegebenenfalls in Gegenwart eines Säurebindemittels umsetzt; daneben gibt es noch andere Herstellungsverfahren. Die Wirkstoffe sind für den Gebrauch als Pflanzenschutzmittel geeignet und mit besonders gutem Erfolg zur Bekämpfung von Oomyceten, z.B. gegen den Erreger der Kraut- und Braunfäule der Tomate und Kartoffel eingesetzt worden.

EP 0 056 161 A2

0056161

BAYER AKTIENGESELLSCHAFT 5090 Leverkusen, Bayerwerk

Zentralbereich
Patente, Marken und Lizenzen Slr-by-kl-c

# Hydroximsäureester, Verfahren zu ihrer Herstellung sowie ihre Verwendung als Fungizide

Die vorliegende Erfindung betrifft neue Hydroximsäureester, mehrere Verfahren zu ihrer Herstellung und ihre Verwendung als Fungizide.

Es ist bereits bekannt geworden, daß Halogenacetanilide, wie beispielsweise N-Chloracetyl-N-(2-ethyl-6-methylphenyl)-alaninmethylester, mit gutem Erfolg zur Bekämpfung von pilzlichen Pflanzenkrankheiten eingesetzt werden können (vergleiche DE-OS 23 50 944). Deren Wirkung ist jedoch, insbesondere bei niedrigen Aufwandmengen und -konzentrationen, insbesondere auch bei der Bekämpfung von Phytophthora-Arten, nicht immer ganz befriedigend.

Es wurden neue Hydroximsäureester der allgemeinen Formel

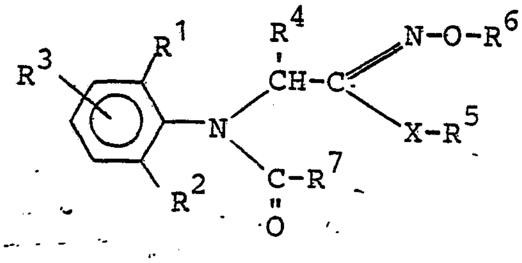

(I)

in welcher

$R^1$ für Wasserstoff, Alkyl und Alkoxy steht,

$R^2$ und $R^3$ für Wasserstoff, Alkyl, Alkoxy und Halogen stehen,

$R^4$ für Wasserstoff und Alkyl steht,

$R^5$ für Alkyl, Alkenyl, Alkinyl, Alkoxyalkyl und Halogenalkenyl steht,

$R^6$ für Wasserstoff, Alkyl, Alkenyl, Alkinyl, Alkoxyalkyl und Halogenalkenyl steht, und weiterhin noch

$R^4$ und $R^5$ sowie die Reste $R^5$ und $R^6$ gemeinsam unter Ringschluß für eine Alkylenbrücke stehen können, und

$R^7$ für Furyl, Tetrahydrofuryl, Thiophenyl und Tetrahydrothiophenyl, für gegebenenfalls durch Cyano oder Thiocyano substituiertes Alkyl, Alkenyl und Alkinyl steht, ferner für gegebenenfalls substituiertes Aryl, Cycloalkyl und Cycloalkenyl steht, für Halogenalkyl mit mehr als einem Halogenatom und schließlich für die Gruppierungen

$-CH_2-Az$, $-CH_2-OR^8$, $-CH_2-SR^8$, $-OR^8$, $-SR^8$, $-CH_2-OSO_2R^8$, $-COOR^8$, $-CH_2-Ar$ und

$-CH_2-O-$ 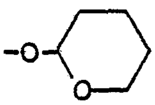 steht, wobei

$R^8$ für gegebenenfalls substituiertes Alkyl, Alkenyl, Alkinyl und Alkoxyalkyl steht,

Az für Pyrazol-1-yl, 1,2,4-Triazol-1-yl und Imidazol-1-yl steht,

Ar für gegebenenfalls substituiertes Phenyl steht, und

X für Sauerstoff oder Schwefel steht,

sowie für den Fall, daß $R^7$ für die Gruppe $-CH_2-Az$ steht, auch deren physiologisch verträglichen Säureadditions-Salze und Metallsalz-Komplexe, gefunden.

Die Verbindungen der Formel I können in der syn- oder anti-Form vorliegen; vorwiegend fallen sie als Gemische beider Formen an.

Weiterhin wurde gefunden, daß man die Hydroximsäureester der Formel I erhält, wenn man

a) Anilin-Derivate der Formel

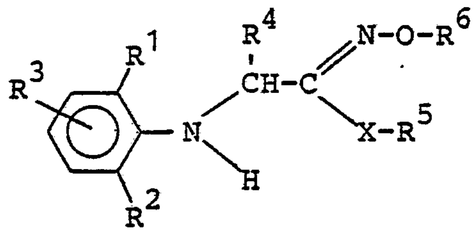

(II)

in welcher

$R^1, R^2, R^3, R^4, R^5$, $R^6$ und X die oben angegebene Bedeutung haben,

mit Säurechloriden oder -bromiden bzw. -anhydriden der Formeln

$$R^7 - \underset{\overset{\|}{O}}{C} - Cl(Br) \qquad (IIIa)$$

bzw.

$$(R^7 - \underset{\overset{\|}{O}}{C} -)_2O \qquad (IIIb)$$

in welcher

$R^7$ die oben angegebene Bedeutung hat,

in Gegenwart eines Verdünnungsmittels und gegebenenfalls in Gegenwart eines Säurebindemittels umsetzt, oder

b) Acylamino-hydroximsäureester der Formel

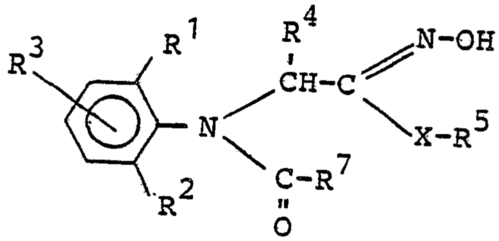

(IV)

in welcher

$R^1$, $R^2$, $R^3$, $R^4$, $R^5$, $R^7$ und X die oben angegebene Bedeutung haben,

gegebenenfalls in Form ihrer Metallsalze mit Alkylierungsmitteln der Formel

$$Y - R^6 \qquad (V)$$

in welcher

$R^6$ die obengenannte Bedeutung, ausgenommen Wasserstoff, besitzt und

Y für Chlor, Brom oder die Gruppe $R^6-SO_2-O$ steht,

in Gegenwart eines organischen Verdünnungsmittels oder in Gegenwart eines organisch-wäßrigen Zweiphasensystems in Gegenwart eines Phasentransfer-Katalysators umsetzt, oder

c) Acylanilide der Formel

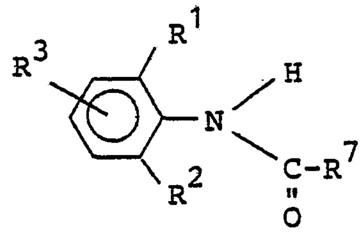

(VI)

in welcher

$R^1$,$R^2$,$R^3$ und $R^7$ die oben angegebene Bedeutung haben,

mit substituierten Alkylhydroximsäureestern der Formel

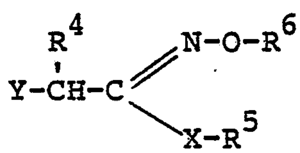

(VII)

in welcher

$R^4$,$R^5$,$R^6$,X und Y die oben angegebene Bedeutung haben,

in Gegenwart eines Säurebindemittels und gegebenenfalls in Gegenwart eines Verdünnungsmittels umsetzt oder

d) für den Fall, daß $R^5$ und $R^6$ gemeinsam unter Ringschluß eine Alkylenbrücke bilden,

Hydroxamsäuren der Formel

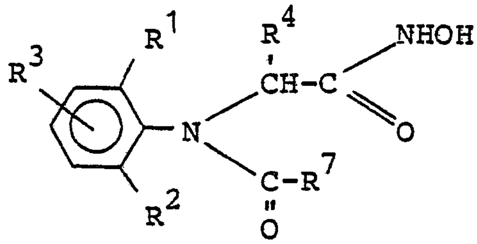

(VIII)

in welcher

$R^1, R^2, R^3, R^4$ und $R^7$ die oben angegebene Bedeutung haben,

in Gegenwart eines Verdünnungsmittels und in Gegenwart eines säurebindenden Mittels mit Verbindungen der Formel

$$Y - (CH_2)_n - Y \qquad (IX)$$

in welcher

Y die obengenannte Bedeutung besitzt und

n eine ganze Zahl von 1 bis 4 darstellt,

umsetzt, oder

e) Halogenacetanilide der Formel

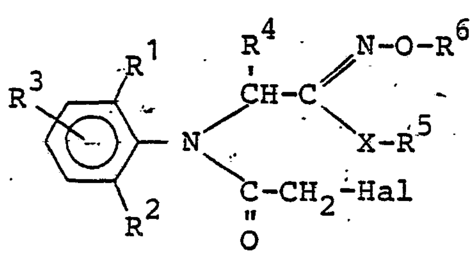

(X)

in welcher

$R^1, R^2, R^3, R^4, R^5, R^6$ und X die oben angegebene Bedeutung haben und

Hal für Chlor, Brom oder Jod steht,

mit Verbindungen der Formel

$$B - R^9 \qquad (XI)$$

in welcher

B für Wasserstoff oder ein Alkalimetall steht und

$R^9$ für Az und die Gruppierungen $-OR^8$ oder $-SR^8$ steht, wobei

Az und $R^8$ die obengenannte Bedeutung besitzen,

in Gegenwart eines Verdünnungsmittels und gegebenenfalls in Gegenwart eines Säurebinders umsetzt.

Die neuen N-Oximinoalkyl-anilide weisen starke fungizide Eigenschaften auf. Dabei zeigen überraschenderweise die erfindungsgemäßen Verbindungen eine erheblich höhere Wirkung als der aus dem Stand der Technik bekannte N-Chloracetyl-N-(2-ethyl-6-methylphenyl)-alaninester,

welcher chemisch und wirkungsmäßig eine naheliegende Verbindung ist. Die erfindungsgemäßen Stoffe stellen somit eine Bereicherung der Technik dar.

Die erfindungsgemäßen N-Oximinoalkyl-anilide sind durch die Formel I allgemein definiert. In dieser Formel stehen $\underline{R}^1$, $\underline{R}^2$, $\underline{R}^3$ und $\underline{R}^4$ vorzugsweise für Wasserstoff und geradkettiges oder verzweigtes Alkyl mit 1 bis 4 Kohlenstoffatomen, $\underline{R}^1$, $\underline{R}^2$ und $\underline{R}^3$ stehen außerdem vorzugsweise für Alkoxy-Gruppen mit 1 bis 4 Kohlenstoffatomen im Alkylteil, ferner zusätzlich noch $\underline{R}^2$ und $\underline{R}^3$ für Halogen, wie insbesondere für Fluor, Chlor und Brom. $\underline{R}^5$ und $\underline{R}^6$ stehen vorzugsweise für Alkyl, Alkenyl und Alkinyl mit jeweils bis zu 4 Kohlenstoffatomen, ferner für Alkoxyalkyl mit jeweils 1 bis 4 Kohlenstoffatomen in den Alkylteilen und für Halogenalkenyl mit 3 bis 4 Kohlenstoffatomen und bis zu 3 Halogenatomen; außerdem kann $\underline{R}^6$ noch zusätzlich vorzugsweise für Wasserstoff stehen. Außerdem können noch $\underline{R}^4$ und $\underline{R}^5$ sowie $\underline{R}^5$ und $\underline{R}^6$ gemeinsam unter Ringschluß für eine Alkylenbrücke mit 1 bis 4 Kohlenstoffatomen stehen.

$\underline{R}^7$ steht vorzugsweise für Furyl, Tetrahydrofuryl, Thiophenyl, Tetrahydrothiophenyl, für gegebenenfalls durch Cyano oder Thiocyano substituiertes Alkyl mit 1 bis 4 und Alkenyl sowie Alkinyl mit jeweils 2 bis 4 Kohlenstoffatomen; ferner vorzugsweise für Dihalogenalkyl mit 1 bis 2 Kohlenstoffatomen, wobei als Halogenatome vorzugsweise Fluor und Chlor genannt seien,

ferner vorzugsweise für Phenyl, welches gegebenenfalls durch Halogen, Alkyl mit 1 bis 3 Kohlenstoffatomen und Methoxy-Gruppen substituiert sein kann, und für Cycloalkyl mit 3 bis 7 Kohlenstoffatomen, sowie für die Gruppierungen $-CH_2-Az$, $-CH_2-OR^8$, $-CH_2-SR^8$, $-OR^8$, $-SR^8$, $-CH_2-OSO_2R^8$, $-COOR^8$ und $-CH_2-O-$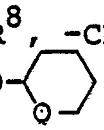 .

Az steht dabei vorzugsweise für Pyrazol-1-yl, 1,2,4-Triazol-1-yl und Imidazol-1-yl. $R^8$ steht vorzugsweise für gegebenenfalls durch Halogen, insbesondere Fluor, Chlor und Brom, Cyano und Thiocyano substituiertes Alkyl mit 1 bis 4 und Alkenyl sowie Alkinyl mit jeweils 2 bis 4 Kohlenstoffatomen und für Alkoxyalkyl mit 1 bis 4 Kohlenstoffatomen in jedem Alkylteil.

Ganz besonders bevorzugt sind diejenigen Hydroximsäureester der allgemeinen Formel I, in denen $R^1$, $R^2$ und $R^3$ für Wasserstoff, Methyl, Ethyl, Isopropyl, sek.-Butyl und tert.-Butyl, ferner für Methoxy, Ethoxy und Isopropoxy stehen, und $R^2$ und $R^3$ noch zusätzlich für Fluor, Chlor und Brom stehen, $R^4$ für Wasserstoff, Methyl und Ethyl steht, $R^5$ und $R^6$ für Methyl, Ethyl, Propyl, Butyl, Isopropyl, Allyl, Methallyl, Monochlor-, Dichlor- und Trichlor-allyl, Propargyl, Methoxymethyl, Ethoxymethyl und Methoxyethyl stehen und $R^6$ noch zusätzlich für Wasserstoff steht, und schließlich $R^4$ und $R^5$ oder $R^5$ und $R^6$ jeweils gemeinsam für eine Methylen-, Ethylen- oder Propylen-Brücke stehen, und

$R^7$ für 2-Furyl, 2-Thienyl, 2-Tetrahydrofuryl, Methoxymethyl, Ethoxymethyl, Allyloxymethyl, Propargyloxymethyl, Methoxyethoxymethyl, Methylmercaptomethyl, Methoxy, Ethoxy, Methylmercapto, Methylsulfonyloxymethyl, Methoxycarbonyl, Ethoxycarbonyl, Methylvinyl, Dichlormethyl, Cyclopropyl, Cyclohexyl, Phenyl, Pyrazol-1-yl-methyl, Imidazol-1-yl-methyl, 1,2,4-Triazol-1-yl-methyl und Tetrahydropyran-2-yl-oxymethyl steht.

Im einzelnen seien außer den bei den Herstellungsbeispielen genannten Verbindungen die folgenden Verbindungen der allgemeinen Formel

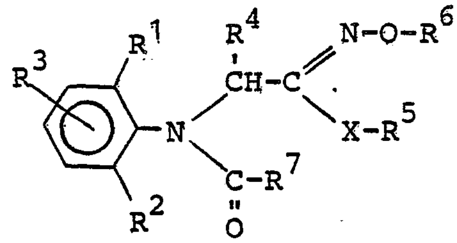

(I)

genannt, wobei

$R^1$ jeweils für Methyl und

$R^3$ für Wasserstoff stehen:

| $R^2$ | $R^4$ | $R^5$ | $R^6$ | $R^7$ | X |
|---|---|---|---|---|---|
| $CH_3$ | $CH_3$ | $CH_3$ | $CH_3$ | $CH_2OCH_3$ | S |
| $CH_3$ | $CH_3$ | $CH_3$ | $C_2H_5$ | $CH_2OCH_3$ | S |
| $CH_3$ | $CH_3$ | $CH_3$ | $C_3H_7$ | $CH_2OCH_3$ | S |
| $CH_3$ | $CH_3$ | $CH_3$ | $CH_2-CH=CH_2$ | $CH_2OCH_3$ | S |
| $CH_3$ | $CH_3$ | $CH_3$ | $C_2H_5$ | $CH_2OCH_3$ | O |
| $CH_3$ | $CH_3$ | $CH_3$ | $-CH_2-CH=CH_2$ | $CH_2OCH_3$ | O |
| $CH_3$ | $CH_3$ | $CH_3$ | $-CH_2-C\equiv CH$ | $CH_2OCH_3$ | O |
| $CH_3$ | $CH_3$ | $C_2H_5$ | $CH_3$ | $CH_2OCH_3$ | O |
| $CH_3$ | $CH_3$ | $C_2H_5$ | $C_2H_5$ | $CH_2OCH_3$ | O |
| Cl | $CH_3$ | $CH_3$ | $CH_3$ | $CH_2OCH_3$ | O |
| Cl | $CH_3$ | $CH_3$ | $CH_3$ | $CH_2OCH_3$ | S |
| $CH_3$ | $CH_3$ | $CH_3$ | $CH_3$ | 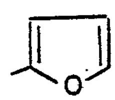 | O |
| $CH_3$ | $CH_3$ | $CH_3$ | $CH_3$ | 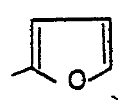 | S |
| $CH_3$ | $CH_3$ | $CH_3$ | $CH_3$ | $-CH_2OCH_2CH_2OCH_3$ | O |

| $R^2$ | $R^4$ | $R^5$ | $R^6$ | $R^7$ | X |
|---|---|---|---|---|---|
| $CH_3$ | $CH_3$ | $CH_3$ | $CH_3$ | $-CH_2OCH_2CH_2OCH_3$ | S |
| $CH_3$ | $CH_3$ | $CH_3$ | $CH_3$ | $-CH_2OCH_2-C \equiv CH$ | O |
| $CH_3$ | $CH_3$ | $CH_3$ | $CH_3$ | $-CH_2OCH_2-C \equiv CH$ | S |
| $CH_3$ | $CH_3$ | $CH_3$ | $CH_3$ | 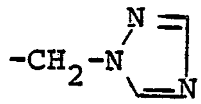 | O |
| $CH_3$ | $CH_3$ | $CH_3$ | $CH_3$ | 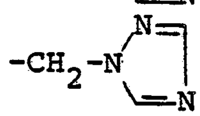 | S |
| $CH_3$ | $CH_3$ | $CH_3$ | $CH_3$ | $-CH=CH-CH_3$ | O |
| $CH_3$ | $CH_3$ | $CH_3$ | $CH_3$ | $-CH=CH-CH_3$ | S |
| $CH_3$ | $CH_3$ | $-CH_2-CH_2-$ | | $-CH_2OCH_3$ | O |
| Cl | $CH_3$ | $-CH_2-CH_2-$ | | $-CH_2OCH_3$ | O |
| $CH_3$ | $CH_3$ | $-CH_2-CH_2-$ | | 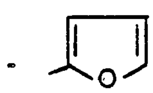 | O |
| $CH_3$ | $CH_3$ | $-CH_2-CH_2-$ | | $-CH=CH-CH_3$ | O |
| $CH_3$ | $CH_3$ | $-CH_2-CH_2-$ | | $-CH_2OCH_2CH_2OCH_3$ | O |
| $CH_3$ | $-CH_2-CH_2-$ | | $CH_3$ | $CH_2OCH_3$ | O |
| $CH_3$ | $-CH_2-CH_2-$ | | $CH_3$ | $CH_2OCH_3$ | S |

| $R^2$ | $R^4$ $R^5$ | $R^6$ | $R^7$ | X |
|---|---|---|---|---|
| $CH_3$ | $-CH_2-CH_2-CH_2-$ | $CH_3$ | $CH_2OCH_3$ | O |
| $CH_3$ | $-CH_2-CH_2-CH_2-$ | $CH_3$ | $CH_2OCH_3$ | S |
| $CH_3$ | $-CH_2-CH_2-$ | $CH_3$ | 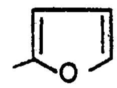 | O |
| $CH_3$ | $-CH_2-CH_2-$ | $CH_3$ | 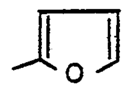 | S |
| $CH_3$ | $-CH_2-CH_2-$ | $CH_3$ | $-CH_2OCH_2CH_2OCH_3$ | O |
| $CH_3$ | $-CH_2-CH_2-$ | $CH_3$ | $-CH_2OCH_2CH_2OCH_3$ | S |

Verwendet man beispielsweise 2,6-Dimethylanilino-acethydroximsäure-O,O'-dimethylester und Methoxyessigsäurechlorid als Ausgangsstoffe, so kann der Reaktionsablauf durch das folgende Formelschema wiedergegeben werden (Verfahren a):

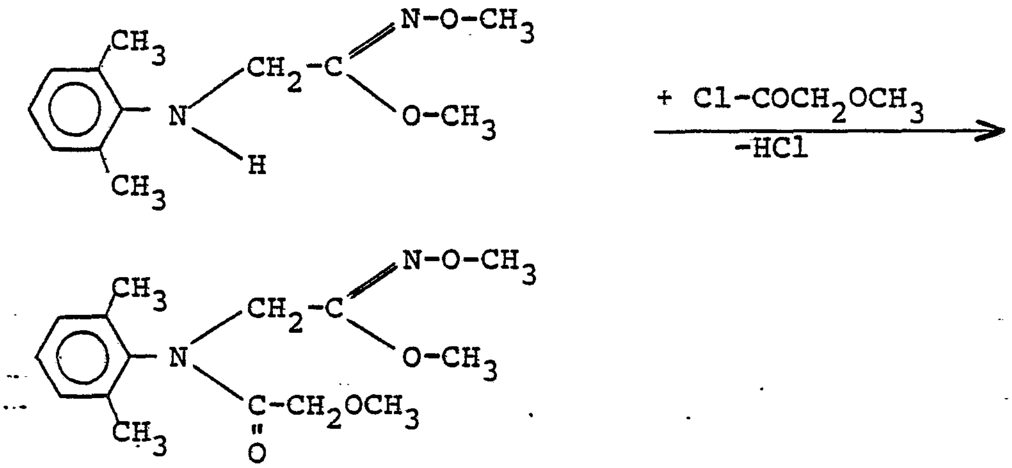


Verwendet man beispielsweise N-2,6-Dimethylphenyl-N-methoxyacetylglycinhydroximsäure-methylester und Dimethylsulfat als Ausgangsstoffe, so kann der Reaktionsablauf durch das folgende Formelschema wiedergegeben werden (Verfahren b):

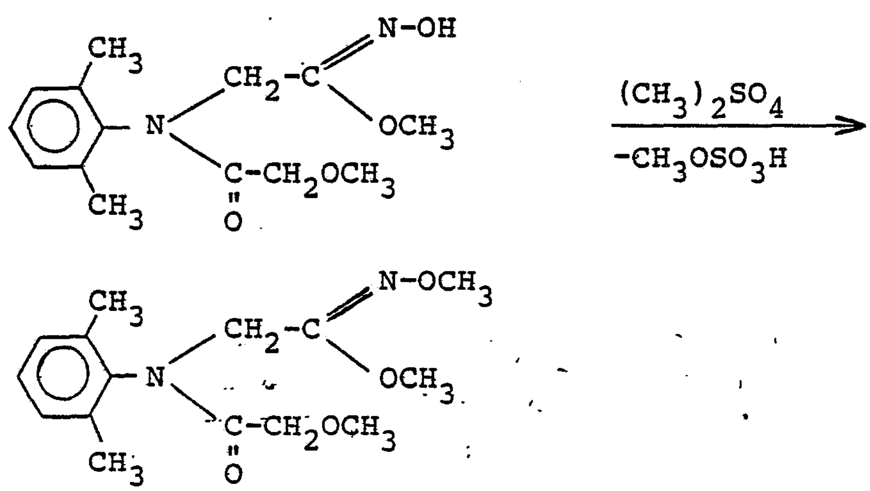

Verwendet man beispielsweise 2,6-Dimethyl-N-(2-furoyl)-anilin und 2-Brom-propionhydroximsäure-O,O'-dimethylester als Ausgangsstoffe, so kann der Reaktionsablauf durch das folgende Formelschema wiedergegeben werden (Verfahren c):

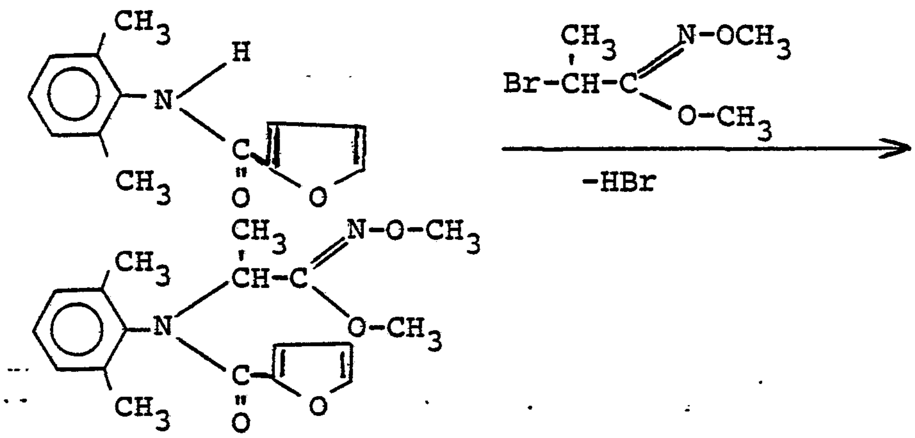


Verwendet man beispielsweise N-2,6-Dimethylphenyl-N-crotonoylanilino-acethydroximsäure und 1,2-Dibromethan als Ausgangsstoffe, so kann der Reaktionsablauf durch das folgende Formelschema wiedergegeben werden (Verfahren d):

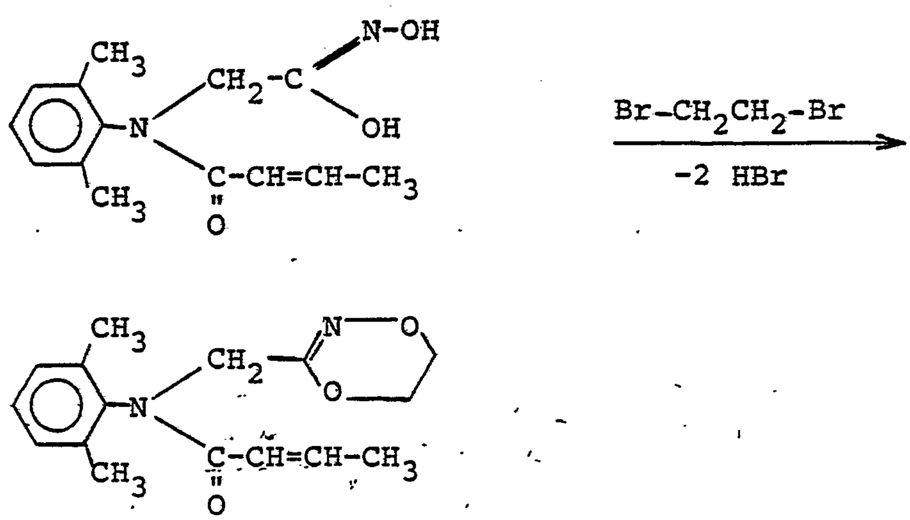

Verwendet man beispielsweise N-(2',6'-Dimethylphenyl)-N-chloracetylalanin-hydroximsäure-O,O'-dimethylester und Imidazol als Ausgangsstoffe, so kann der Reaktionsablauf durch das folgende Formelschema wiedergegeben werden (Verfahren e):

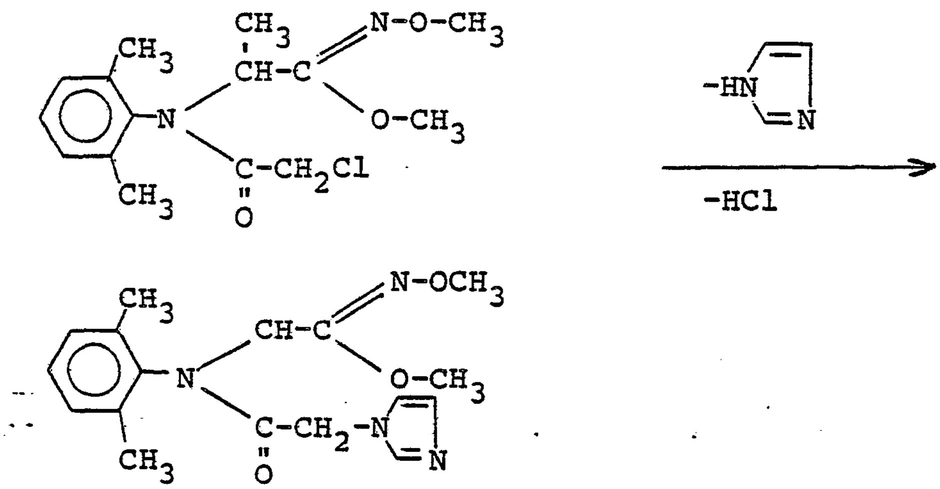


Die bei der Durchführung des erfindungsgemäßen Verfahrens a) als Ausgangsstoffe benötigten Anilin-Derivate sind durch die Formel II allgemein definiert. In dieser Formel stehen $R^1$, $R^2$, $R^3$, $R^4$, $R^5$, $R^6$ und X vorzugsweise für diejenigen Reste, die bereits im Zusammenhang mit der Beschreibung der erfindungsgemäßen Stoffe der Formel I vorzugsweise für diese Reste genannt wurden.

Die Anilin-Derivate der Formel II sind noch nicht bekannt. Sie können erhalten werden, indem man z.B. Aniline der Formel

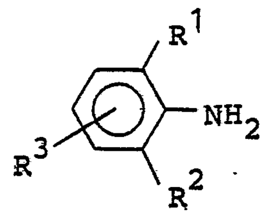

(XII)

in welcher

$R^1$, $R^2$ und $R^3$ die oben angegebene Bedeutung besitzen,

mit substituierten Alkylhydroximsäureestern der obengenannten Formel VII in Gegenwart eines Säurebinders, wie beispielsweise Alkalicarbonate, und gegebenenfalls in Gegenwart eines inerten organischen Lösungsmittels wie beispielsweise Toluol oder Dimethylformamid, bei Temperaturen zwischen 20 und 160°C, vorzugsweise zwischen 60 und 100°C umsetzt.

Die als weitere Ausgangsstoffe benötigten Aniline der Formel XII sind allgemein bekannte Verbindungen der organischen Chemie. Als Beispiele seien genannt: 2-Methylanilin, 2,6-Dimethylanilin; 2,3,6-Trimethylanilin, 2,4,6-Trimethylanilin, 2-Methyl-6-ethylanilin; 2,6-Diethyl-anilin; 2-Chlor-6-methyl-anilin; 2-Brom-6-methylanilin; 2-Fluor-6-methylanilin; 2-Methoxyanilin; 2-Methoxy-2-methylanilin; 2,6-Dimethoxyanilin.

Die weiterhin als Vorprodukte benötigten substituierten Alkylhydroximsäureester der Formel VII (diese Verbindungen

stellen auch Ausgangsstoffe für die Verfahrensvariante c) dar) sind teilweise bekannt (vgl. GB-PS 1 275 806). Sie können erhalten werden, indem man Halogen-hydroximsäure-Derivate der Formel

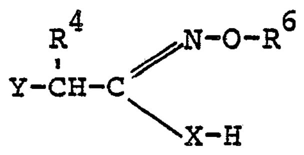 (XIII)

in welcher

$R^4$, $R^6$, X und Y die weiter oben angegebene Bedeutung besitzen,

mit Diazoalkanen der Formel

$$N_2=R^{5a} \quad (XIV)$$

in welcher

$R^{5a}$ die Bedeutung von $R^5$ hat, jedoch an der Verknüpfungsstelle ein Wasserstoffatom weniger aufweist,

in einem inerten Lösungsmittel, wie Ether oder Toluol, bei Temperaturen zwischen 0 und +50°C umsetzt; oder nach einem bisher noch nicht beschriebenen Verfahren, indem man Verbindungen der Formel

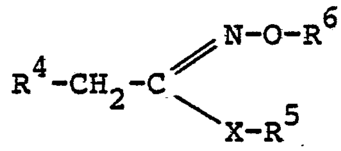

(XV)

in welcher

$R^4$, $R^5$, $R^6$ und X die oben angegebene Bedeutung besitzen,

mit üblichen Halogenierungsmitteln (wie z.B. Chlor, Brom oder N-Halogen-succinimid) in einem inerten Lösungsmittel wie beispielsweise Methylenchlorid, Chloroform oder Tetrachlorkohlenstoff, vorzugsweise unter Belichten und Zugabe eines Radikalbildners, oder unter Zusatz eines Säurebinders wie beispielsweise Pyridin oder Alkalicarbonat, bei Temperaturen zwischen 0 und 100°C umsetzt; oder indem man $\alpha$-Hydroxy-hydroximsäureester der Formel

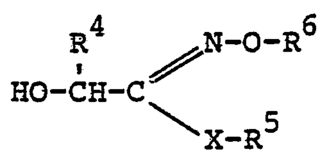

(XVI)

in welcher

$R^4$,$R^5$,$R^6$ und X die weiter oben angegebene Bedeutung besitzen,

mit gebräuchlichen Halogenierungsmitteln wie Thionylchlorid, Phosphortrichlorid, Phosphortribromid und Thionylbromid in einem inerten Lösungsmittel wie beispielsweise Methylenchlorid, Toluol oder Diethylether, gegebenenfalls unter Zusatz eines Säurebinders wie Pyridin oder Alkalicarbonat, umsetzt.

Die Halogen-hydroximsäure-Derivate der Formel XIII sind teilweise bekannt (vgl. GB-PS 1 275 806). Sie können erhalten werden, indem man Halogencarbonsäure-halogenide der Formel

$$\text{Y-}\underset{}{\overset{R^4}{\overset{|}{C}}}\text{H-CO-Z} \qquad \text{(XVII)}$$

in welcher

$R^4$ und Y die oben angegebene Bedeutung besitzen und

Z für Halogen steht,

mit Hydroxylaminen bzw. deren Salzen der Formel

$$H_2N\text{-}O\text{-}R^6 \qquad \text{(XVIII)}$$

in welcher

$R^6$ die oben angegebene Bedeutung besitzt,

in einem inerten Lösungsmittel wie Toluol oder Methylenchlorid, gegebenenfalls unter Zusatz eines Säurebindemittels, wie Pyridin oder eines anorganischen Carbonats wie Alkalicarbonat, bei Temperaturen zwischen 0 und +50°C, vorzugsweise zwischen 0 und 30°C, umsetzt.

Die Verbindungen der Formel XV sind teilweise bekannt. Sie können erhalten werden, indem man Hydroximsäureester der Formel

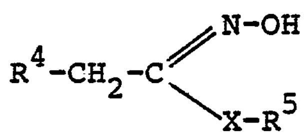

(XIX)

in welcher

$R^4$, $R^5$ und X die oben angegebene Bedeutung besitzen,

mit Alkylierungsmitteln der Formel V

$Y-R^6$ (V)

in welcher

$R^6$ die oben genannte Bedeutung besitzen und

Y für Chlor, Brom oder die Gruppe $R^6-SO_2-O$ steht,

in Gegenwart eines organischen Lösungsmittels, wie Aceton oder Dimethylformamid, oder in Gegenwart eines organisch-wäßrigen Zweiphasensystems in Gegenwart eines Phasen-Transfer-Katalysators, wie z.B.

Triethyl-benzyl-ammoniumchlorid, bei Temperaturen zwischen 0 und +100°C, vorzugsweise zwischen 0 und 50°C umsetzt.

Die Hydroximsäureester der Formel XIX sind bekannt (zur Herstellung vgl. Houben-Weyl, "Methoden der organischen Chemie", Band VIII, Seiten 686 ff., Georg Thieme Verlag, Stuttgart (1952)); zu ihrer Herstellung kann man beispielsweise Alkylhalogenide auf alkalische Lösungen von Hydroxamsäuren einwirken lassen und spaltet das Reaktionsprodukt selektiv.

Die α-Hydroxy-hydroximsäureester der Formel XVI sind noch nicht bekannt; sie können erhalten werden, indem man Ketohydroximsäureester der Formel

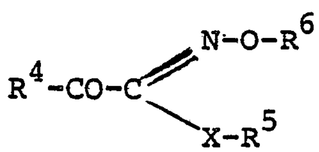

(XX)

in welcher

$R^4$, $R^5$, $R^6$ und X die oben angegebene Bedeutung besitzen,

mit gebräuchlichen Reduktionsmitteln, wie beispielsweise Natriumborhydrid oder Aluminiumisopropylat, in organischen Lösungsmitteln wie Methanol oder Dioxan bei Temperaturen zwischen 0 und +80°C, vorzugsweise zwischen 20 und 60°C reduziert. Die Ketohydroximsäure-Derivate der Formel XX sind ebenfalls

neu. Sie können erhalten werden, indem man Keto-hydroximsäure-Derivate der Formel

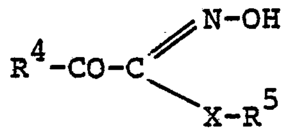

(XXI)

in welcher

$R^4$, $R^5$ und X die oben angegebene Bedeutung besitzen,

mit Alkylierungsmitteln der Formel (V)

$Y-R^6$ (V)

in welcher

$R^6$ die obengenannte Bedeutung besitzt und

Y für Chlor, Brom oder die Gruppe $R^6-SO_2-O$ steht,

in Gegenwart eines organischen Lösungsmittels wie beispielsweise Aceton oder Dimethylformamid oder in Gegenwart eines wäßrig-organischen Zweiphasensystems wie Natronlauge-Methylenchlorid oder Natronlauge-Toluol in Gegenwart eines Phasen-Transfer-Katalysators, wie z.B. Triethyl-benzyl-ammoniumchlorid, bei Temperaturen zwischen 0 und +100°C, vorzugsweise zwischen 0 und 50°C, umsetzt.

Die Keto-hydroximsäure-Derivate der Formel XXI sind teilweise bekannt (vgl. DE-OS 21 11 459); sie können auch erhalten werden, wenn man Ketone der Formel

$$R^4\text{-CO-CH}_2\text{-X-R}^5 \qquad \text{(XXII)}$$

in welcher

$R^4$,$R^5$ und X die obengenannte Bedeutung besitzen,

mit Nitrosierungsmitteln, wie z.B. iso-Amylnitrit, in Methanol als Verdünnungsmittel unter Zusatz äquimolarer Mengen Natrium-methylat umsetzt.

Die Ketone der Formel XXII sind teilweise bekannt (vgl. DE-OS 22 16 838). Sie können erhalten werden, wenn man Halogenketone der Formel

$$R^4\text{-CO-CH}_2\text{-Hal} \qquad \text{(XXIII)}$$

in welcher

$R^4$ die oben angegebene Bedeutung besitzt und

Hal für Halogen steht,

mit den Alkalisalzen von Alkoholen oder Mercaptanen der Formel

$$Me\text{-}X\text{-}R^5 \qquad (XXIV)$$

in welcher

$R^5$ und X die oben angegebene Bedeutung besitzen und

Me für ein Äquivalent eines Kations, vorzugsweise $Na^{(+)}$ steht,

zur Umsetzung bringt.

Die bei der Durchführung der erfindungsgemäßen Verfahrensvariante b) als Ausgangsstoffe benötigten Acylamino-hydroximsäureester sind durch die allgemeine Formel IV bezeichnet. In dieser Formel stehen $R^1$, $R^2$, $R^3$, $R^4$, $R^5$ und $R^7$ vorzugsweise für diejenigen Reste, die bereits im Zusammenhang mit der Beschreibung der erfindungsgemäßen Stoffe der Formel I vorzugsweise für diese Reste genannt wurden.

Die Acylamino-hydroximsäureester der Formel IV sind noch nicht bekannt. Sie können erhalten werden, indem man z.B. Nitrile der Formel

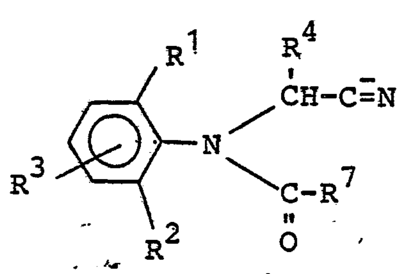

(XXV)

in welcher

$R^1, R^2, R^3, R^4, R^7$ und X die oben angegebene Bedeutung haben,

mit Hilfe der sogenannten "Pinner-Reaktion" (vgl. Ber. Dtsch. Chem. Ges. 16, 356 (1883)) mit Alkoholen der Formel $R^5$-OH und Chlorwasserstoff-Gas in Salze der Formel

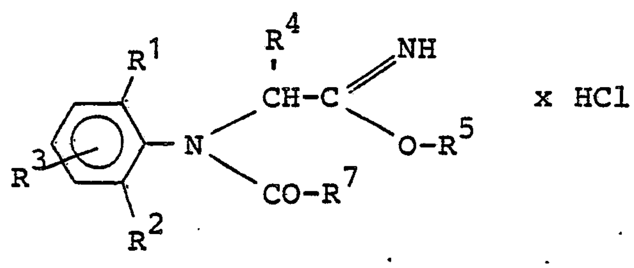

(XXVI)

in welcher

$R^1, R^2, R^3, R^4, R^5$ und $R^7$ die oben angegebene Bedeutung besitzen,

überführt und diese Salze nach üblichen Verfahren (vgl. Ber. Dtsch. Chem. Ges. 17, 184 (1884)) mit Hydroxylamin-Hydrochlorid zu den Verbindungen der Formel IV umsetzt.

Die Nitrile der Formel XXV sind aus den entsprechenden N-Phenyl-glycinonitril-Derivaten der Formel

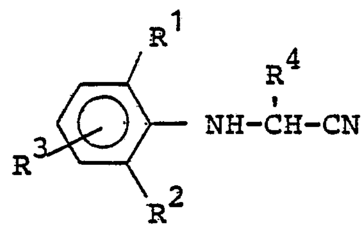

(XXVII)

in welcher

$R^1$ bis $R^4$ die oben angegebene Bedeutung besitzen,

(welche letztere z.B. in allgemein bekannter Weise aus einem Anilin der Formel XII, Aldehyd und Cyanwasserstoff hergestellt werden können) und dem entsprechenden Säurechlorid der Formel

$$Cl\text{-}CO\text{-}R^7 \qquad (XXVIII)$$

in welcher

$R^7$ die oben angegebene Bedeutung besitzt,

in Gegenwart eines Säureakzeptors erhalten.

Die bei der Durchführung der erfindungsgemäßen Verfahrensvariante c) als Ausgangsstoffe benötigten Acylanilide der Formel VI (siehe oben) sind bekannt (vgl. DE-OS 28 47 287.9 (Le A 19 217)). Die Herstellung der weiterhin als Ausgangsstoffe benötigten Alkylhydroximsäureester der Formel VII ist oben beschrieben.

Die bei der Durchführung der erfindungsgemäßen Verfahrensvariante d) als Ausgangsstoffe benötigten Hydroxamsäuren sind durch die Formel VIII allgemein definiert. In dieser Formel stehen $R^1$, $R^2$, $R^3$, $R^4$ und $R^7$ vorzugsweise für diejenigen Reste die bereits im Zusammenhang mit der Beschreibung der erfindungsgemäßen Stoffe der Formel (I) vorzugsweise für diese Reste genannt wurden.

Die Hydroxamsäuren der Formel VIII sind ebenfalls fungizid wirksam. Sie sind Gegenstand einer noch nicht veröffentlichten Anmeldung (Deutsche Patentanmeldung P 30 33 160.3 vom 3.9.1980 (Le A 20 512)). Sie werden hergestellt, indem man z.B. substituierte Aniline der Formel

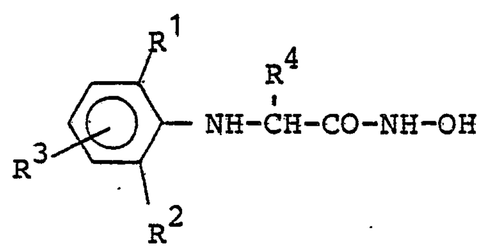 (XXIX)

in welcher

$R^1$ bis $R^4$ die weiter oben angegebene Bedeutung besitzen,

mit einem Säurechlorid der Formel XXVIII

$$Cl-CO-R^7$$

in welcher

$R^7$ die oben angegebene Bedeutung besitzt,

in Gegenwart eines Verdünnungsmittels und in Gegenwart eines Säurebindemittels bei etwa +20 bis 100°C umsetzt. Die substituierten Aniline der Formel XXIX sind noch nicht bekannt. Sie werden erhalten, indem man z.B. Aniline der Formel XII

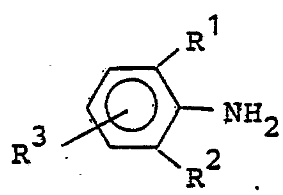

(XII)

in welcher

$R^1$, $R^2$ und $R^3$ die oben angegebene Bedeutung besitzen,

mit substituierten Oxiamiden der Formel

$$\begin{array}{c} R^4 \\ | \\ Y-CH-CO-NH-OH \end{array} \qquad (XXX)$$

in welcher

Y und $R^4$ die oben angegebene Bedeutung besitzen,

in Gegenwart eines Säurebinders, wie beispielsweise von Alkalicarbonaten, und gegebenenfalls in Gegenwart eines inerten organischen Lösungsmittels, wie z.B. Toluol, bei Temperaturen zwischen +20 und 160°C umsetzt. Die dazu benötigten substituierten Oxiamide der Formel XXX können aus α-Halogencarbonsäurechloriden der Formel

$$Y-\overset{\displaystyle R^4}{\overset{|}{C}}H-CO-Cl \qquad (XXXI)$$

und Hydroxylamin leicht hergestellt werden.

Die Hydroxamsäuren der Formel (VIII) lassen sich auch erhalten, indem man die bekannten entsprechenden Carbonsäurealkylester (vgl. z.B. US-P 4 098 895) gegebenenfalls in Gegenwart eines Lösungsmittels, vorzugsweise Methanol oder Ethanol, und gegebenenfalls in Gegenwart einer Base, wie Kaliumhydroxid oder Natriumhydroxid, mit Hydroxylamin bei Temperaturen zwischen 0°C und 60°C umsetzt.

Die für die Herstellung gemäß Verfahrensvariante d) weiterhin benötigten Ausgangsstoffe der Formel IX sind allgemein bekannte Verbindungen der organischen Chemie.

Die für die Herstellung gemäß Verfahrensvariante e) als Ausgangsstoffe benötigten Halogenacetanilide sind durch die Formel X allgemein definiert. In dieser Formel stehen $R^1$, $R^2$, $R^3$, $R^4$, $R^5$, $R^6$ und X vorzugsweise für diejenigen Reste, die bereits bei der Beschreibung der erfindungsgemäßen Verbindungen der Formel I vorzugsweise für diese Reste genannt wurden.

Die Verbindungen der Formel X sind selbst fungizid und selektiv herbizid wirksam (sie sind Gegenstand der Deutschen Patentanmeldung P 30 42 243.6 vom 8.11.1980 (Le A 20 640)). Sie werden hergestellt, indem man die Anilin-Derivate der Formel II (siehe oben, allgemeine Beschreibung von Verfahren a) mit Halogenessigsäure-chloriden oder -bromiden der Formel

$$(Br)Cl-CO-CH_2-Hal \qquad (XXXII)$$

in Gegenwart eines Verdünnungsmittels und gegebenenfalls in Gegenwart eines Säurebindemittels umsetzt, z.B. im Temperaturbereich zwischen 0 und 50°C.

Die für das Verfahren gemäß Variante e) weiterhin benötigten Verbindungen der Formel XI sind allgemein bekannte Verbindungen der organischen Chemie.

Als Verdünnungsmittel kommen für die erfindungsgemäße Umsetzung gemäß Verfahren a) vorzugsweise inerte organische Lösungsmittel in Frage. Hierzu gehören vorzugsweise Ketone, wie Diethylketon, insbesondere Aceton und Methylethylketon; Nitrile, wie Propionitril, insbesondere Acetonitril; Ether, wie Tetrahydrofuran oder Dioxan, aliphatische und aromatische Kohlenwasserstoffe, wie Petrolether, Benzol, Toluol oder Xylol; halogenierte Kohlenwasserstoffe, wie Methylenchlorid, Tetrachlorkohlenstoff, Chloroform oder Chlorbenzol; und Ester, wie Essigester.

Das erfindungsgemäße Verfahren a) kann gegebenenfalls in Gegenwart von Säurebindern (Halogenwasserstoff-Akzeptoren) durchgeführt werden. Als solche können alle üblichen Säurebindemittel verwendet werden. Hierzu gehören vorzugsweise organische Basen, wie tertiäre Amine, beispielsweise Triethylamin, oder Pyridin, ferner anorganische Basen, wie beispielsweise Alkalihydroxide und Alkalicarbonate.

Die Reaktionstemperaturen können bei der Durchführung des erfindungsgemäßen Verfahrens (a) in einem größeren Bereich variiert werden. Im allgemeinen arbeitet man zwischen -20°C und +120°C, vorzugsweise zwischen 0 und 60°C.

Bei der Durchführung des erfindungsgemäßen Verfahrens a) setzt man vorzugsweise auf 1 Mol der Verbindungen der Formel II 1 bis 1,5 Mol Acylierungsmittel und 1 bis 1,5 Mol Säurebinder ein. Die Isolierung der Verbindungen der Formel I erfolgt in üblicher Weise.

Als Verdünnungsmittel kommen für die erfindungsgemäße Umsetzung gemäß Verfahren b) inerte organische Lösungsmittel in Frage.

Vorzugsweise zu nennen sind Ether, wie Diethylether, aromatische Kohlenwasserstoffe, wie z.B. Toluol, chlorierte Kohlenwasserstoffe, wie Methylenchlorid, und schließlich Dimethylformamid.

Setzt man die Verbindungen der Formel IV in Form ihrer Alkalisalze ein, so erfolgt die Darstellung der letzteren in situ durch Zugabe äquimolarer Mengen an Alkalialkoholat oder -hydrid.

Die Reaktionstemperaturen können beim Verfahren b) in einem größeren Bereich variiert werden, im allgemeinen arbeitet man zwischen -20 und +100°C, vorzugsweise zwischen 0 und 80°C.

Gemäß einer bevorzugten Ausführungsform führt man die Umsetzung b) als Zweiphasen-Reaktion durch unter Verwendung von wäßriger Natronlauge und Methylenchlorid oder Toluol unter Zusatz eines Phasen-Transfer-Katalysators, wie z.B. Triethylbenzyl-ammoniumchlorid.

Als Verdünnungsmittel kommen für die erfindungsgemäße Umsetzung gemäß Verfahren (c) vorzugsweise inerte organische Lösungsmittel in Frage. Hierzu gehören vorzugsweise die bei dem Verfahren (a) bereits genannten Solventien.

Als Säurebindemittel kommen bei dem Verfahren (c) vorzugsweise die bei dem Verfahren (a) bereits genannten Stoffe in Frage.

Die Reaktionstemperaturen können bei der Durchführung des erfindungsgemäßen Verfahrens (c) in einem größeren Bereich variiert werden. Im allgemeinen arbeitet man zwischen -20°C und +150°C, vorzugsweise zwischen 0°C und 100°C.

Bei der Durchführung des erfindungsgemäßen Verfahrens (c) arbeitet man vorzugsweise mit äquimolaren Mengen.

Es ist jedoch auch möglich, eine der Komponenten in einem Überschuß einzusetzen. Die Aufarbeitung und Isolierung der Reaktionsprodukte erfolgen nach üblichen Methoden.

In einer bevorzugten Ausführungsform wird die erfindungsgemäße Umsetzung gemäß Verfahren (c) in einem Zweiphasensystem, wie beispielsweise wäßrige Natron- oder Kalilauge/Toluol oder Methylenchlorid, gegebenenfalls unter Zusatz von 0,1 bis 1 Mol eines Phasen-transfer-Katalysators, wie beispielsweise einer Ammonium- oder Phosphoniumverbindung, beispielsweise seien Benzyl-dodecyl-dimethyl-ammoniumchlorid und Triethyl-benzyl-ammoniumchlorid genannt, durchgeführt.

Als Verdünnungsmittel kommen für die erfindungsgemäße Umsetzung gemäß Verfahren (d) alle inerten organischen Lösungsmittel in Frage. Hierzu gehören vorzugsweise Ether, wie Diethylether und Dioxan; aromatische Kohlenwasserstoffe, wie Toluol und Benzol; Alkohole, wie Methanol oder Ethanol; in einzelnen Fällen auch chlorierte Kohlenwasserstoffe, wie Chloroform, Methylenchlorid und Tetrachlorkohlenstoff; sowie Hexamethylphosphorsäure-triamid und Dimethylformamid.

Die Reaktionstemperaturen können bei der Durchführung des erfindungsgemäßen Verfahrens (d) in einem größeren Bereich variiert werden. Im allgemeinen arbeitet man zwischen -20 und +150°C, vorzugsweise bei Raumtemperatur. In einzelnen Fällen ist es vorteilhaft, bei der

Siedetemperatur des Lösungsmittels, beispielsweise zwischen 60 und 100°C, zu arbeiten.

Bei der Durchführung des erfindungsgemäßen Verfahrens (d) setzt man auf 1 Mol Alkalisalz der Formel (VIII) vorzugsweise 1 bis 3 Mol an Verbindung der Formel (IX) ein. Die Aufarbeitung und Isolierung der Reaktionsprodukte erfolgen nach üblichen Methoden.

Die Reaktionstemperaturen können beim Verfahren (e) in einem größeren Bereich variiert werden. Im allgemeinen arbeitet man zwischen etwa 20 bis 150°C, vorzugsweise bei 60 bis 120°C. Bei Anwesenheit eines Lösungsmittels wird zweckmäßigerweise beim Siedepunkt des jeweiligen Lösungsmittels gearbeitet.

Bei der Durchführung des erfindungsgemäßen Verfahrens (e) setzt man auf 1 Mol der Verbindungen der Formel (X) vorzugsweise 1 bis 2 Mol der Verbindungen der Formel (XI) und gegebenenfalls 1 bis 2 Mol Säurebinder ein. Die Isolierung der Verbindungen der Formel (I) erfolgt in üblicher Weise.

Die erfindungsgemäßen Wirkstoffe weisen eine starke mikrobizide Wirkung auf und können zur Bekämpfung von unerwünschten Mikroorganismen praktisch eingesetzt werden. Die Wirkstoffe sind für den Gebrauch als Pflanzenschutzmittel geeignet.

Fungizide Mittel im Pflanzenschutz werden eingesetzt zur Bekämpfung von Plasmodiophoromycetes, Oomycetes, Chytridiomycetes, Zygomycetes, Ascomycetes, Basidiomycetes, Deuteromycetes.

Die gute Pflanzenverträglichkeit der Wirkstoffe in den zur Bekämpfung von Pflanzenkrankheiten notwendigen Konzentrationen erlaubt eine Behandlung von oberirdischen Pflanzenteilen, von Pflanz- und Saatgut, und des Bodens.

Als Pflanzenschutzmittel können die erfindungsgemäßen Wirkstoffe mit besonders gutem Erfolg zur Bekämpfung von Oomyceten, z.B. gegen den Erreger der Kraut- und Braunfäule der Tomate und Kartoffel (Phytophthora infestans), eingesetzt werden. Weiterhin liegt eine Wirkung gegen Erreger von Reiskrankheiten vor, z.B. gegen Pyricularia oryzae und Pellicularia sasakii.

Die Wirkstoffe können in die üblichen Formulierungen übergeführt werden, wie Lösungen, Emulsionen, Suspensionen, Pulver, Schäume, Pasten, Granulate, Aerosole, Wirkstoff-imprägnierte Natur- und synthetische Stoffe, Feinstverkapselungen in polymeren Stoffen und in Hüllmassen für Saatgut, ferner in Formulierungen mit Brennsätzen, wie Räucherpatronen, -dosen, -spiralen u.ä., sowie ULV-Kalt- und Warmnebel-Formulierungen.

Diese Formulierungen werden in bekannter Weise hergestellt, z.B. durch Vermischen der Wirkstoffe mit Streckmitteln, also flüssigen Lösungsmitteln, unter Druck stehenden verflüssigten Gasen und/oder festen Trägerstoffen, gegebenenfalls unter Verwendung von oberflächenaktiven Mitteln, also Emulgiermitteln und/oder Dispergiermitteln und/oder schaumerzeugenden Mitteln. Im Falle der Benutzung von Wasser als Streckmittel können z.B. auch organische Lösungsmittel als Hilfslösungsmittel verwendet werden. Als flüssige Lösungsmittel kommen im wesentlichen in Frage: Aromaten, wie Xylol, Toluol, oder Alkylnaphthaline, chlorierte Aromaten oder chlorierte aliphatische Kohlenwasserstoffe, wie Chlorbenzole, Chloräthylene oder Methylenchlorid, aliphatische Kohlenwasserstoffe, wie Cyclohexan oder Paraffine, z.B. Erdölfraktionen, Alkohole, wie Butanol oder Glycol sowie deren Äther und Ester, Ketone, wie Aceton, Methyläthylketon, Methylisobutylketon oder Cyclohexanon, stark polare Lösungsmittel, wie Dimethylformamid und Dimethylsulfoxid, sowie Wasser; mit verflüssigten gasförmigen Streckmitteln oder Trägerstoffen sind solche Flüssigkeiten gemeint, welche bei normaler Temperatur und unter Normaldruck gasförmig sind, z.B. Aerosol-Treibgas, wie Halogenkohlenwasserstoffe sowie Butan, Propan, Stickstoff und Kohlendioxid; als feste Trägerstoffe kommen in Frage: z.B. natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate; als feste Trägerstoffe für Granulate kommen in Frage: z.B. gebrochene und fraktionierte natürliche Gesteine wie

Calcit, Marmor, Bims, Sepiolith, Dolomit sowie synthetische Granulate aus anorganischen und organischen Mehlen sowie Granulate aus organischem Material wie Sägemehl, Kokosnußschalen, Maiskolben und Tabakstengel; als Emulgier- und/oder schaumerzeugende Mittel kommen in Frage: z.B. nichtionogene und anionische Emulgatoren, wie Polyoxyäthylen-Fettsäure-Ester, Polyoxyäthylen-Fettalkohol-Äther, z.B. Alkylarylpolyglykol-äther, Alkylsulfonate, Alkylsulfate, Arylsulfonate sowie Eiweißhydrolysate; als Dispergiermittel kommen in Frage: z.B. Lignin-Sulfitablaugen und Methylcellulose.

Es können in den Formulierungen Haftmittel wie Carboxymethylcellulose, natürliche und synthetische pulverige, körnige und latexförmige Polymere verwendet werden, wie Gummiarabicum, Polyvinylalkohol, Polyvinylacetat.

Es können Farbstoffe wie anorganische Pigmente, z.B. Eisenoxid, Titanoxid, Ferrocyanblau und organische Farbstoffe, wie Alizarin-, Azol-Metallphthalocyaninfarbstoffe und Spurennährstoffe wie Salze von Eisen, Mangan, Bor, Kupfer, Kobalt, Molybdän und Zink verwendet werden.

Die Formulierungen enthalten im allgemeinen zwischen 0,1 und 95 Gewichtsprozent Wirkstoff, vorzugsweise zwischen 0,5 und 90 %.

Die erfindungsgemäßen Wirkstoffe können in den Formulierungen oder in den verschiedenen Anwendungsformen in Mischung mit anderen bekannten Wirkstoffen vorliegen, wie Fungiziden, Bakteriziden, Insektiziden, Akariziden, Nematiziden, Herbiziden, Schutzstoffen gegen Vogelfraß, Wuchsstoffen, Pflanzennährstoffen und Bodenstrukturverbesserungsmitteln.

Die Wirkstoffe können als solche, in Form ihrer Formulierungen oder der daraus durch weiteres Verdünnen bereiteten Anwendungsformen, wie gebrauchsfertige Lösungen, Emulsionen, Suspensionen, Pulver, Pasten und Granulate angewendet werden. Die Anwendung geschieht in üblicher Weise, z.B. durch Gießen, Tauchen, Spritzen, Sprühen, Vernebeln, Verdampfen, Injizieren, Verschlämmen, Verstreichen, Stäuben, Streuen, Trockenbeizen, Feuchtbeizen, Naßbeizen, Schlämmbeizen oder Inkrustieren.

Bei der Behandlung von Pflanzenteilen können die Wirkstoffkonzentrationen in den Anwendungsformen in einem größeren Bereich variiert werden. Sie liegen im allgemeinen zwischen 1 und 0,0001 Gew.-%, vorzugsweise zwischen 0,5 und 0,001 %.

Bei der Saatgutbehandlung werden im allgemeinen Wirkstoffmengen von 0,001 bis 50 g je Kilogramm Saatgut, vorzugsweise 0,01 bis 10 g, benötigt.

Bei Behandlung des Bodens sind Wirkstoffkonzentrationen von 0,00001 bis 0,1 Gew.-%, vorzugsweise von 0,0001 bis 0,02 %, am Wirkungsort erforderlich.

Herstellungsbeispiele

Beispiel 1

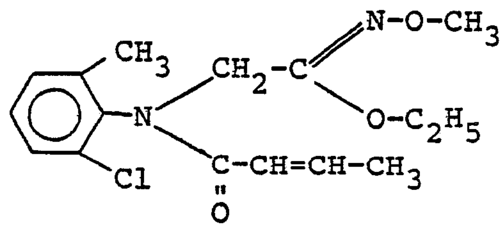

(Verfahren a))

Zu einer Lösung von 6 g (0,023 Mol) O-Ethyl-N-(2-chlor-6-methyl-phenyl)-glycin-hydroximsäure-methylester (siehe unten) in 50 ml Dichlormethan und 3 g (0,038 Mol) Pyridin werden bei 0°C 3 g (0,029 Mol) Crotonsäurechlorid getropft. Man rührt einige Stunden nach, läßt über Nacht stehen und wäscht dann 2 mal mit je 20 ml Wasser. Nach Trocknen mit Natriumsulfat und Einengen im Vakuum wird der Rückstand im Kugelrohr destilliert (Kp. 0,2 mm Hg/160°C) und das Destillat mit wenig Diisopropylether verrieben. Man erhält 4,1 g N-Crotonoyl-O-ethyl-N-(2-chlor-6-methyl-phenyl)-glycinhydroximsäuremethylester vom Fp. 64-66°C, das sind 54 % der Theorie.

Herstellung der Vorprodukte

VP 1.1

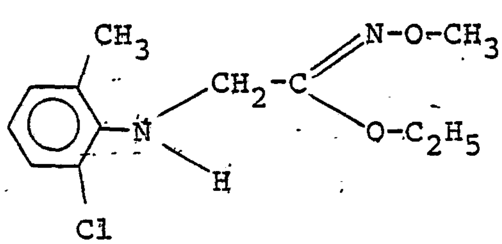

90 g (0,64 Mol) 2-Chlor-6-methylanilin werden in 160 ml Acetonitril mit 30 g (0,22 Mol) fein zerriebenem Kaliumcarbonat und 1 g Kaliumjodid unter Rückfluß gerührt. Innerhalb 1 Stunde tropft man 30 g (0,22 Mol) Ethylchloracethydroxamsäure, gelöst in 100 ml Acetonitril, zu und rührt weitere 2 Stunden bei Siedetemperatur weiter. Nach Abfiltrieren, Auswaschen des Rückstandes mit Dichlormethan und Einengen der flüssigen Phase im Vakuum (0,5 mbar, Bad 120°C) wird der Rückstand in 50 ml Alkohol gelöst und bei 0°C zu einer Lösung von 11,2 g (0,2 Mol) Kaliumhydroxid in 100 ml Alkohol getropft, danach bei gleicher Temperatur 25,2 g (0,2 Mol) Dimethylsulfat. Nach einstündigem Rühren bei Raumtemperatur wird für 30 Minuten auf 50°C erwärmt und danach im Vakuum der Alkohol abdestilliert. Der Rückstand wird in Dichlormethan aufgenommen, zweimal mit Wasser ausgewaschen, mit Natriumsulfat getrocknet und nach Abziehen des Lösungsmittels im Vakuum destilliert. Man erhält 24,5 g (47,7 %) Ethyl-N-(2-chlor-6-methyl-phenyl)-glycin-hydroxamsäuremethylester im Siedebereich 132-145°C bei 10 bis 12 mbar mit einem Brechungsindex von $n_D^{20}$ 1,5265.

VP 1.2

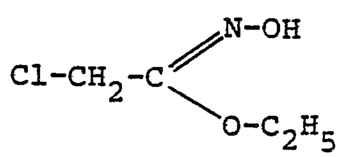

158 g (1 Mol) Imino-chloressigsäureethylester-hydrochlorid werden bei 0°C in eine Lösung von 276 g Kaliumcarbonat (2 Mol) in 500 ml Wasser eingetragen und 10 Minuten kräftig durchgerührt. Danach ethert man sofort 5 mal aus und trägt die vereinigten Etherphasen unter Eiskühlung schnell in eine Lösung von 140 g (2 Mol) Hydroxylamino-hydrochlorid in 250 ml Wasser ein. 15 Minuten wird bei 0°C kräftig gerührt, danach die organische Phase abgetrennt und nach Nachextraktion mit 4 mal je 100 ml Ether über Natriumsulfat getrocknet und im Vakuum eingeengt. Das auskristallisierende Produkt wird abgesaugt und man erhält 64 g (46,5 %) Ethylchloracethydroxamsäure vom Fp. 64-65°C.

Beispiel 2

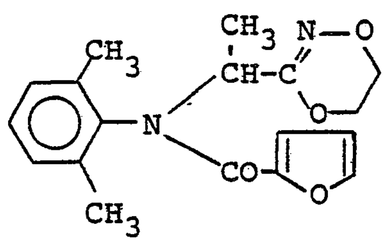

(Verfahren a))

5 g (0,021 Mol) 3-/α-(2,6-Dimethylanilino)-ethyl/-1,4-diox-2-azin, 1,8 g Pyridin (0,023 Mol) und 50 ml Dichlormethan werden auf 0°C gekühlt, mit 4 g (0,031 Mol) Furancarbonsäurechlorid versetzt und über Nacht bei

20°C stehengelassen. Dann wird die Lösung zweimal mit Sodalösung und mit Wasser gewaschen, mit Natriumsulfat getrocknet und im Vakuum eingeengt. Es verbleiben 4 g 3-[α-(N-Furoyl-2,6-dimethylanilino)-ethyl]-1,4-diox-2-azin mit dem Brechungsindex $n_D^{20}$ 1,5567, das sind 58 % der Theorie.

<u>Herstellung der Vorprodukte</u>

VP 2.1

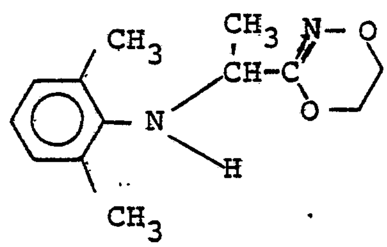

19 g (0,1 Mol) 3-(α-Bromethyl)-1,4-diox-2-azin werden mit 30 g 2,6-Dimethylanilin (0,25 Mol) und 14 g (0,1 Mol) feinzerriebenem Kaliumcarbonat unter Stickstoff 8 Stunden auf 130°C erhitzt. Nach Zusatz von 100 ml Dichlormethan wird danach vom Salz abfiltriert und das Lösungsmittel im Vakuum wieder entfernt. Die Destillation im Vakuum ergibt beim Kp 0,28 mbar/ 135-140°C 16,8 g 3-[α-(2,6-Dimethylanilino)-3-ethyl-1,4-diox-2-azin mit dem Brechungsindex $n_D^{20}$ = 1,5451, das sind 73 % der Theorie.

VP 2.2

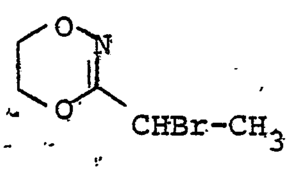

34,5 g (0,3 Mol) 3-Ethyl-1,4-diox-2-azin und 54 g (0,3 Mol) N-Bromsuccinimid werden in 300 ml Tetrachlorkohlenstoff bei Belichtung 75 Minuten unter Rückfluß erhitzt. Nach Filtration und Einengen im Vakuum wird der Rückstand destilliert. Man erhält bei Kp 0,13 mbar/95-100°C 44,8 g 3-(α-Bromethyl)-1,4-diox-2-azin vom Brechungsindex $n_D^{20}$ = 1,5086, das sind 77 % der Theorie.

VP 2.3 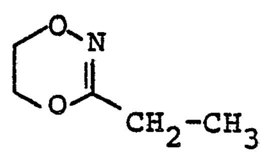

105 g (1,5 Mol) Hydroxylamin-hydrochlorid, gelöst in 660 ml Methanol werden bei 40°C mit 168 g (3 Mol) Kaliumhydroxid, in 650 ml Methanol gelöst, versetzt. Dann gibt man 132 g (1,5 mol) Propionsäuremethylester zu, läßt über Nacht stehen und filtriert danach das ausgefallene Kaliumchlorid ab. Die Mutterlauge wird mit 207 g (1,5 Mol) Kaliumcarbonat unter Rühren auf 40°C erwärmt und nach tropfenweiser Zugabe von 282 g (1,5 Mol) 1,2-Dibromethan 30 Stunden bei gleicher Temperatur gehalten. Danach filtriert man, extrahiert die Salzphase noch mehrfach mit Ether und engt die organischen Anteile ein. Destillation im Vakuum ergibt 65,1 g 3-Ethyl-1,4-diox-2-azin vom Kp 16 mbar/73-76°C und einem Brechungsindex $n_D^{20}$= 1,4520. Die Ausbeute beträgt 38 % der Theorie.

Beispiel 3

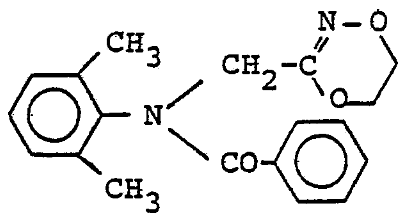

(Verfahren d))

15 g (0,22 Mol) Hydroxylamin-hydrochlorid werden in 80 ml Methanol gelöst und bei 40°C zu einer Lösung von 16,8 g (0,3 Mol) Kaliumhydroxid in 42 ml Methanol gegeben. Nach Filtration werden sofort 31 g (0,1 Mol) N-Benzoyl-N-(2,6-dimethylphenyl)-glycinethylester, gelöst in 50 ml Methanol, zugesetzt und über Nacht stehengelassen. Dann gibt man 200 ml Methanol, 13,8 g (0,1 Mol) Kaliumcarbonat und 18,8 g (0,1 Mol) Dibromethan zu, rührt 30 Stunden bei 50°C, dampft dann ein, nimmt in 10 %iger Natronlauge und Ether auf und trennt die Etherphase schnell ab. Nach Trocknen über Natriumsulfat und Abziehen der leichtflüchtigen Bestandteile im Vakuum verbleiben ca. 20 g eines hellen Öls, das auf ca. 100 g Kieselgel durch Säulenchromatographie weiter gereinigt werden kann. Elution mit Cyclohexan/Aceton steigender Polarität (je 0,5 l einer Mischung im Verhältnis von 100:1, 50:1, 20:1, 10:1, 5:1, 2:1) ergibt ein farbloses Öl, das bald erstarrt. Ebenso kristallisiert das rohe Öl beim Verreiben mit Diisopropylether.

Bei chromatographischer Reinigung ergeben 15 g Öl 6 g (das sind 25 % der Theorie) an 3-[α-(N-Benzoyl-2,6-dimethylanilino)-methyl]-1,4-diox-2-azin vom Fp. 98°C.

Beispiel 4

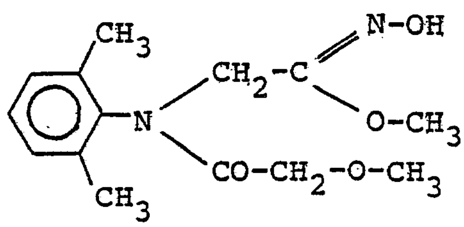

(Verfahren b))

25 g (0,11 Mol) N-Methoxyacetyl-N-(2,6-dimethyl-phenyl)-aminoessigsäurenitril werden in 100 ml Dichlormethan und 3,5 g (0,11 Mol) Methanol gelöst. Bei 0°C leitet man trockenen Chlorwasserstoff ein, bis ein farbloses Salz ausfällt, das nach Abfiltrieren im Vakuum getrocknet wird (hygroskopisch, ca. 30 g). Danach trägt man es bei 0°C in eine Lösung von 15 g Kaliumcarbonat (0,11 Mol) in 40 ml Wasser ein, rührt kurz durch und extrahiert schnell 3 mal mit je 40 ml Ether. Die vereinigten Etherphasen läßt man bei 0°C in eine Lösung von 20 g (0,29 Mol) Hydroxylamin-hydrochlorid in 30 ml Wasser einlaufen, rührt 10 Minuten kräftig, und trennt wieder ab. Nach dreimaliger Nachextraktion, Trocknen mit Natriumsulfat und Abdestillieren des

Lösungsmittels verbleibt ein farbloses Öl. Man erhält 6 g (das sind 21 % der Theorie) N-Methoxyacetyl-N-(2,6-dimethyl-phenyl)-glycin-methylhydroxamsäure, die beim Verreiben mit Diisopropylether Kristalle vom Fp. 103°C ergibt.

Beispiel 5

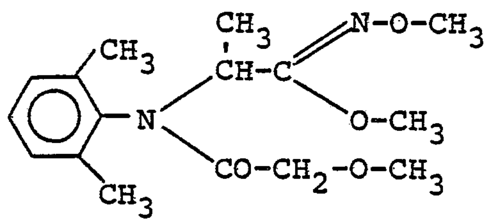

(Verfahren a))

3 g α-(2,6-Dimethylanilino)-propionhydroxamsäure-O,O'-dimethylester und 1,1 g Pyridin werden in 50 ml absolutem Toluol gelöst und bei 70°C tropfenweise mit einer Lösung von 1,5 g Methoxyacetylchlorid versetzt. Es wird noch 2 Stunden bei 70°C gerührt, die organische Phase nach Abkühlen mit Wasser gewaschen und dann im Vakuum vom Lösungsmittel befreit. Das resultierende Öl wird im Kugelrohr destilliert (Kp 0,1 mmHg/140°C).

Herstellung der Vorprodukte

VP 5.1 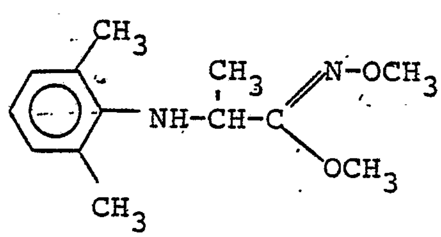

11 g (0,092 Mol) 2,6-Dimethylanilin und 6,4 g (0,046 Mol) fein gepulvertes Kaliumcarbonat werden in 30 ml Dimethylformamid bei 80°C unter Rühren tropfenweise mit 9 g (0,046 Mol) 2-Brom-propionhydroximsäure-O,O'-dimethylester versetzt. Es wird noch 5 Stunden bei 80°C gerührt und danach der anorganische Niederschlag abfiltriert. Nach Abdestillieren von Dimethylformamid und der Hauptmenge an 2,6-Dimethylanilin wird der ölige Rückstand am Kugelrohr destilliert. Der Siedepunkt liegt bei Kp 0,1 mmHg/100°C. Die Ausbeute beträgt 3,3 g.

VP 5.2 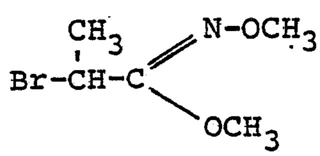

8,2 g (0,07 Mol) Propionhydroximsäure-O,O'-dimethylester und 12,46 g (0,07 Mol) N-Brom-succinimid werden in 100 ml Tetrachlorkohlenstoff unter Belichten mit einer 300 Watt-Lampe 4 Stunden am Rückfluß gekocht. Anschließend wird das abgeschiedene Succinimid abfiltriert und das Filtrat am Wasserstrahlvakuum destilliert. Der Siedepunkt liegt bei Kp 12 mmHg/65-75°C. Die Ausbeute beträgt 7,6 g, das sind 55 % der Theorie.

Entsprechend den Beispielen 1 bis 5 wurden noch die folgenden Verbindungen der allgemeinen Formel

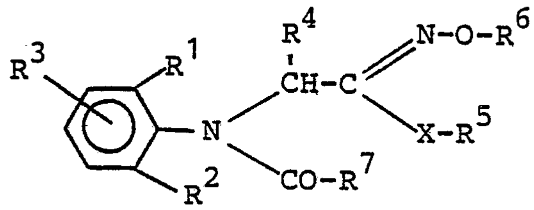

(I)

hergestellt:

| Bsp. Nr. | $R^1$ | $R^2$ | $R^3$ | $R^4$ | $XR^5$ | $R^6$ | $R^7$ | Physikalische Eigenschaften |
|---|---|---|---|---|---|---|---|---|
| 6 | H | $CH_3$ | H | H | $OCH_3$ | $CH_3$ | 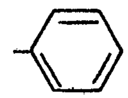 | $n_D^{20}$ = 1,5619 |
| 7 | H | $C_2H_5$ | H | H | $OCH_3$ | $CH_3$ | $CH_2OCH_3$ | $n_D^{20}$ = 1,5139 |
| 8 | H | $OCH_3$ | H | H | $OCH_3$ | $CH_3$ | $CH_2OCH_3$ | $n_D^{20}$ = 1,5259 |
| 9 | H | Cl | H | H | $OC_2H_5$ | $CH_3$ | $CH_2OCH_3$ | $n_D^{20}$ = 1,5112 |
| 10 | $CH_3$ | H | 3-$CH_3$ | H | $OCH_3$ | $CH_2$-CH=$CH_2$ | $CH_2OCH_3$ | $n_D^{20}$ = 1,5201 |
| 11 | $CH_3$ | H | 3-$CH_3$ | H | $OCH_2CH_2CH_3$ | $CH_3$ | $CH_2OCH_3$ | $n_D^{20}$ = 1,5133 |
| 12 | $CH_3$ | H | 3-$CH_3$ | H | $OCH_2CH_2CH_3$ | $CH_2$-CH=$CH_2$ | $CH_2OCH_3$ | $n_D^{20}$ = 1,5171 |
| 13 | $CH_3$ | $CH_3$ | H | H | $OCH_3$ | $CH_3$ | $CH_2OCH_3$ | $n_D^{20}$ = 1,5205 |
| 14 | $CH_3$ | $CH_3$ | H | H | $OCH_3$ | $C_2H_5$ | $CH_2OCH_3$ | $n_D^{20}$ = 1,5138 |
| 15 | $CH_3$ | $CH_3$ | H | H | $OC_2H_5$ | $CH_3$ | $CH_2OCH_3$ | $n_D^{20}$ = 1,5095 |

| Bsp. Nr. | $R^1$ | $R^2$ | $R^3$ | $R^4$ | $XR^5$ | $R^6$ | $R^7$ | Physikalische Eigenschaften |
|---|---|---|---|---|---|---|---|---|
| 16 | $CH_3$ | $CH_3$ | H | H | $OC_2H_5$ | $CH_3$ | $CH=CHCH_3$ | $n_D^{20}$= 1,5272 |
| 17 | $CH_3$ | $CH_3$ | H | H | $OC_2H_5$ | $C_2H_5$ | $CH_2OCH_3$ | $n_D^{20}$= 1,5030 |
| 18 | $CH_3$ | $CH_3$ | H | H | $OC_2H_5$ | $C_2H_5$ | $CH=CH-CH_3$ | Fp. 82-83°C |
| 19 | $CH_3$ | $CH_3$ | H | H | $OCH_2CH_2CH_3$ | $CH_3$ | $CH_2OCH_3$ | $n_D^{20}$= 1,5113 |
| 20 | $CH_3$ | $CH_3$ | H | H | $OCH_2CH_2CH_3$ | $CH_2-CH=CH_2$ | $CH_2OCH_3$ | $n_D^{20}$= 1,5093 |
| 21 | $CH_3$ | $CH_3$ | H | H | $-O-CH_2-CH_2-$ | | $CH_2OCH_3$ | Fp. 63-65°C |
| 22 | $CH_3$ | $CH_3$ | H | $CH_3$ | $-O-CH_2-CH_2-$ | | $CH_2-OCH_3$ | $n_D^{20}$= 1,5068 |
| 23 | $CH_3$ | $CH_3$ | H | $CH_3$ | $-O-CH_2-CH_2-$ | | $CH=CH-CH_3$ | IR: C=O 1735 $cm^{-1}$<br>C=N 1620 $cm^{-1}$ |

| Bsp. Nr. | $R^1$ | $R^2$ | $R^3$ | $R^4$ | $XR^5$ | $R^6$ | $R^7$ | Physikalische Eigenschaften |
|---|---|---|---|---|---|---|---|---|
| 24 | $CH_3$ | Cl | H | H | $OC_2H_5$ | $CH_3$ | $CH_2OCH_3$ | $n_D^{20}$= 1,5117 |
| 25 | $CH_3$ | Cl | H | H | $-O-CH_2-CH_2-$ | | $CH_2OCH_3$ | Fp. 73-74°C |
| 26 | $CH_3$ | Cl | H | $CH_3$ | $-O-CH_2-CH_2-$ | | $CH_2OCH_3$ | $n_D^{20}$= 1,5224 |
| 27 | $CH_3$ | Cl | H | $CH_3$ | $-O-CH_2-CH_2-$ | | $CH=CHCH_3$ | IR: C=O 1730 $cm^{-1}$<br>C=N 1620 $cm^{-1}$ |
| 28 | $C_2H_5$ | $CH_3$ | H | H | $OC_2H_5$ | $CH_3$ | $CH_2OCH_3$ | Kp 0,05 mm/160 |
| 29 | $C_2H_5$ | $CH_3$ | H | H | $OCH_2CH_2CH_3$ | $CH_3$ | $CH_2OCH_3$ | $n_D^{20}$= 1,5109 |
| 30 | $C_2H_5$ | $CH_3$ | H | H | $OCH_2CH_2CH_3$ | $CH_2CH=CH_2$ | $CH_2OCH_3$ | $n_D^{20}$= 1,5080 |
| 31 | $C_2H_5$ | $CH_3$ | H | H | $-O-CH_2-CH_2-$ | | $CH_2OCH_3$ | $n_D^{20}$= 1,5347 |
| 32 | $C_2H_5$ | $C_2H_5$ | H | H | $OC_2H_5$ | $CH_2CH=CH_2$ | $CH_2OCH_3$ | $n_D^{20}$= 1,5056 |

| Bsp. Nr. | $R^1$ | $R^2$ | $R^3$ | $R^4$ | $XR^5$ | $R^6$ | $R^7$ | Physikalische Eigenschaften |
|---|---|---|---|---|---|---|---|---|
| 33 | $C_2H_5$ | $C_2H_5$ | H | H | $OCH_2CH_2CH_3$ | $CH_3$ | $CH_2OCH_3$ | $n_D^{20}$ = 1,5075 |
| 34 | $C_2H_5$ | $C_2H_5$ | H | H | $OCH_2CH_2CH_3$ | $CH_2CH=CH_2$ | 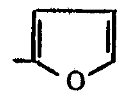 | $n_D^{20}$ = 1,5271 |
| 35 | $C_2H_5$ | $C_2H_5$ | H | H | $-O-CH_2-CH_2-$ | | $CH_2OCH_3$ | $n_D^{20}$ = 1,5315 |
| 36 | $CH_3$ | $C_2H_5$ | H | $CH_3$ | $OCH_3$ | $CH_3$ | $CH_2OCH_3$ | $n_D^{20}$ = 1,5050 |
| 37 | $CH_3$ | $CH_3$ | H | H | $OCH_2CH_3$ | $CH_3$ | 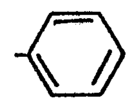 | $n_D^{20}$ = 1,5532 |
| 38 | H | $OCH_3$ | H | H | $OCH_3$ | $C_2H_5$ | $CH_2OCH_3$ | $n_D^{20}$ = 1,5091 |
| 39 | H | $OCH_3$ | H | H | $OCH_3$ | $CH_2-C\equiv CH$ | $CH_2OCH_3$ | $n_D^{20}$ = 1,5200 |
| 40 | H | $CH_3$ | H | H | $OCH_3$ | $C_2H_5$ | $CH_2OCH_3$ | $n_D^{20}$ = 1,5029 |
| 41 | H | $CH_3$ | H | H | $OCH_3$ | $CH_2-C\equiv CH$ | $CH_2OCH_3$ | $n_D^{20}$ = 1,5099 |
| 42 | $CH_3$ | Cl | H | $CH_3$ | $OCH_3$ | $CH_3$ | $CH_2OCH_3$ | $n_D^{20}$ = 1,5320 |

Beispiel 43

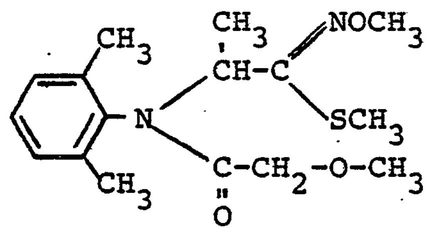

3,5 g (0,014 Mol) 1-Thiomethyl-1-methoximino-2-(2',6'-dimethyl-anilino)-propan, gelöst in 50 ml Dichlormethan und 1,6 g (0,02 Mol) Pyridin, werden auf 0°C gekühlt und tropfenweise mit 2,0 g (0,018 Mol) Methoxyacetylchlorid versetzt. Nach dem Stehen über Nacht wird 30 Minuten unter Rückfluß erhitzt. Danach wird das Reaktionsgemisch mit Wasser versetzt, und die organische Phase abgetrennt. Man wäscht die organische Phase zweimal mit Wasser, trocknet über Natriumsulfat und engt unter vermindertem Druck ein. Man erhält auf diese Weise 4 g (83,9 % der Theorie) der Verbindung obiger Formel in Form eines viskosen Öles mit einem Brechungsindes $n_D^{20}$= 1,5440.

Herstellung der Vorprodukte:

VP 43.1

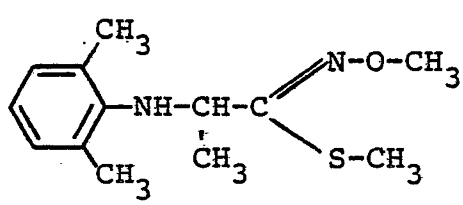

7,0 g (0,042 Mol) 1-Methoximino-1-thiomethyl-2-chlor-

propan, 12 g (0,10 Mol) 2,6-Dimethylanilin, 7 g (0,05 Mol) feingepulvertes Kaliumcarbonat (wasserfrei) und 1 g Natriumjodid werden unter Rühren 10 Stunden auf 150°C erhitzt. Dann nimmt man das Reaktionsgemisch in 100 ml Dichlormethan auf, filtriert die anorganischen Bestandteile ab und destilliert das nach dem Entfernen des Lösungsmittels verbleibende Produkt im Hochvakuum. Man erhält 5 g (47,1 % der Theorie) der Verbindung der obigen Formel in Form eines farblosen Öles mit einem Siedepunkt von 140°C/25 mbar und einem Brechungsindex $n_D^{20}$ = 1,5583.

VP 43.2

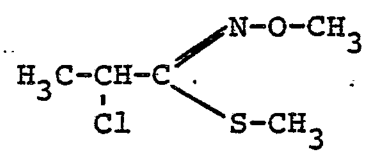

46 g (0,31 Mol) 1-Methoximino-1-thiomethyl-2-hydroxypropan werden in 300 ml Cyclohexan und 26,9 g (0,34 Mol) Pyridin unter Rückfluß und Feuchtigkeitsausschluß erhitzt und tropfenweise mit 40,3 g (0,34 Mol) Thionylchlorid versetzt. Man hält die Temperatur bis zum Ende der Gasentwicklung konstant, versetzt das Reaktionsgemisch dann mit Wasser und trennt die Phasen. Die organische Phase wird mit Wasser gewaschen, unter vermindertem Druck eingeengt, und der verbleibende Rückstand wird destilliert. Man erhält 32,8 g (63,4 % der Theorie) an 1-Methoximino-1-thiomethyl-2-chlorpropan mit einem Brechungsindex von $n_D^{20}$ = 1,5115 und einem Siedepunkt von 120 - 125°C bei 0,35 mbar.

VP 43.3

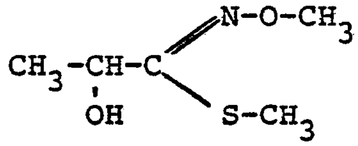

65 g (0,44 Mol) 1-Methoximino-1-thiomethyl-2-oxopropan werden in 200 ml Alkohol gelöst und portionsweise mit insgesamt 25 g (0,66 Mol) Natriumboranat versetzt, wobei sich das Gemisch erwärmt. Man rührt eine Stunde nach, engt dann im Vakuum ein, nimmt in 200 ml Wasser auf und extrahiert fünfmal mit je 50 ml Chloroform. Man trocknet über Natriumsulfat und erhält nach dem Eindampfen durch Destillation des verbleibenden Rückstandes 53 g (80,4 % der Theorie) an 1-Methoximino-1-thiomethyl-2-hydroxy-propan in Form eines viskosen, hellen Öles mit einem Brechungsindex von $n_D^{20}$= 1,5129 und einem Siedepunkt von 100-110°C bei 0,45 mbar.

VP 43.4

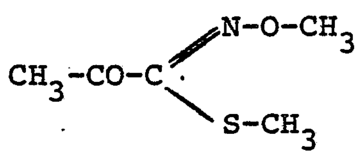

116,5 g (ca. 1 Mol) 1-Oximino-1-thiomethyl-2-oxo-propan wird in eine Lösung von 49 g (0,88 Mol) Kaliumhydroxid in 1 l Methanol eingetragen und 30 Minuten bei Raumtemperatur gerührt. Anschließend werden 121,4 g (0,96 Mol) Dimethylsulfat zugetropft. Danach erhitzt man 30 Minuten unter Rückfluß. Nach dem Verdünnen des Reaktionsgemisches mit Wasser auf das doppelte Volumen wird mit Kalilauge alkalisiert, dreimal mit Chloroform extrahiert, die organische Phase abgetrennt und mit Aktivkohle und Natriumsulfat behandelt. Nach dem Eindampfen verbleiben 65,8 g (51,2 % der Theorie) an 1-Methoximino-1-thiomethyl-2-oxo-propan in Form eines rötlichen Öles mit dem Brechungsindex $n_D^{20}$ = 1,5023.

Vp. 43.5

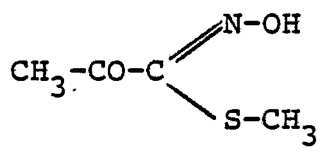

Zu einer Lösung von 30,5 g (1,33 Mol) Natrium in 1 l absolutem Alkohol werden 135 g (1,15 Mol) Isoamylnitrit gegeben; dann werden bei 0°C 125 g (1,2 Mol) Methylmercaptoaceton eingetropft. Die Mischung wird 4 Stunden bei Raumtemperatur nachgerührt. Danach dampft man das Reaktionsgemisch auf das halbe Volumen ein, setzt 500 ml Wasser zu, schüttelt die wäßrige Lösung dreimal mit Petrolether und einmal mit Chloroform aus, säuert dann mit konzentrierter Salzsäure auf pH 5 bis 6 an, extrahiert das Reaktionsprodukt mit dreimal je 100 ml Chloroform, trocknet über Natriumsulfat und dampft das Lösungsmittel ab. Man erhält 116,5 g (76,2 % der Theorie) von 1-Oximino-1-thiomethyl-2-oxo-propan in Form eines hellen Öles vom Brechungsindex $n_D^{20}$ = 1,5168.

Beispiel 44

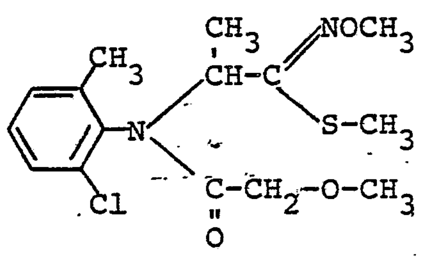

$n_D^{20}$ = 1,5292

Analog den Vorprodukten VP 1.1, 2.1 und 5.1 wurden noch die folgenden Vorprodukte der allgemeinen Formel

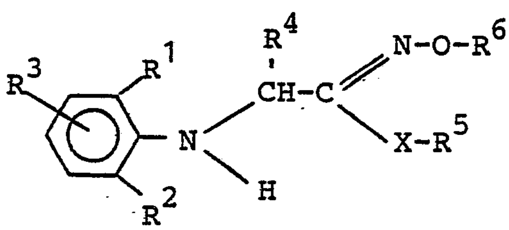

(II)

hergestellt:

| Vorprodukt Nr. | $R^1$ | $R^2$ | $R^3$ | $R^4$ | $XR^5$ | $R^6$ | Kp (mmHg/°C) |
|---|---|---|---|---|---|---|---|
| VP 6 | H | $CH_3$ | H | H | $OCH_3$ | $CH_3$ | 0,3/130 |
| VP 7 | H | $C_2H_5$ | H | H | $OCH_3$ | $CH_3$ | 0,6/114 |
| VP 8 | H | $OCH_3$ | H | H | $OCH_3$ | $CH_3$ | 0,8/130 |
| VP 9 | H | Cl | H | H | $OCH_3$ | $CH_3$ | 0,8/110 |
| VP 10 | $CH_3$ | H | 3-$CH_3$ | H | $OCH_3$ | $CH_2$-CH=$CH_2$ | 0,4/130 |
| VP 11 | $CH_3$ | H | 3-$CH_3$ | H | O-n-$C_3H_7$ | $CH_3$ | 0,1/140 |
| VP 12 | $CH_3$ | H | 3-$CH_3$ | H | O-n-$C_3H_7$ | $CH_2$-CH=$CH_2$ | 1/150 |
| VP 13 | $CH_3$ | $CH_3$ | H | H | $OCH_3$ | $CH_3$ | 0,2/110 |
| VP 14 | $CH_3$ | $CH_3$ | H | H | $OC_2H_5$ | $CH_3$ | 0,6/100 |
| VP 15 | $CH_3$ | $CH_3$ | H | H | $OC_2H_5$ | $CH_3$ | 0,6/100 |

| Vorprodukt Nr. | $R^1$ | $R^2$ | $R^3$ | $R^4$ | $R^5$ | $R^6$ | Kp (mmHg/°C) |
|---|---|---|---|---|---|---|---|
| VP 16 | $CH_3$ | $CH_3$ | H | H | $OC_2H_5$ | $C_2H_5$ | 0,2/120 |
| VP 17 | $CH_3$ | $CH_3$ | H | H | $OCH_2CH_2CH_3$ | $CH_3$ | 0,7/120 |
| VP 18 | $CH_3$ | $CH_3$ | H | H | $OCH_2CH_2CH_3$ | $CH_2CH=CH_2$ | 1/140 |
| VP 19 | $CH_3$ | Cl | H | $CH_3$ | $-O-CH_2-CH_2-$ | | 0,3/140 |
| VP 20 | $C_2H_5$ | $CH_3$ | H | H | $OC_2H_5$ | $CH_3$ | 0,4/115 |
| VP 21 | $C_2H_5$ | $CH_3$ | H | H | $OCH_2CH_2CH_3$ | $CH_3$ | 2/130 |
| VP 22 | $C_2H_5$ | $CH_3$ | H | H | $OCH_2CH_2CH_3$ | $CH_2CH=CH_2$ | 0,1/150 |
| VP 23 | $C_2H_5$ | $C_2H_5$ | H | H | $OC_2H_5$ | $CH_2CH=CH_2$ | 1/130 |
| VP 24 | $C_2H_5$ | $C_2H_5$ | H | H | $OCH_2CH_2CH_3$ | $CH_3$ | 0,1/115 |
| VP 25 | $C_2H_5$ | $C_2H_5$ | H | H | $OCH_2CH_2CH_3$ | $CH_2CH=CH_2$ | 0,1/150 |

| Vorprodukt Nr. | $R^1$ | $R^2$ | $R^3$ | $R^4$ | $R^5$ | $R^6$ | Kp (mmHg/°C) |
|---|---|---|---|---|---|---|---|
| VP 26 | $CH_3$ | $C_2H_5$ | H | $CH_3$ | $OCH_3$ | $CH_3$ | 0,5/100 |
| VP 27 | $CH_3$ | Cl | H | $CH_3$ | $OCH_3$ | $CH_3$ | 0,2/130 |
| VP 28 | H | $OCH_3$ | H | H | $OCH_3$ | $C_2H_5$ | 0,7/150 |
| VP 29 | H | $OCH_3$ | H | H | $OCH_3$ | $CH_2-C\equiv CH$ | 0,5/160 |
| VP 30 | H | $CH_3$ | H | H | $OCH_3$ | $C_2H_5$ | 0,6/150 |
| VP 31 | H | $CH_3$ | H | H | $OCH_3$ | $CH_2-C\equiv CH$ | 0,3/130 |
| VP 32 | $CH_3$ | Cl | H | $CH_3$ | $SCH_3$ | $CH_3$ | 0,3/140 |

Entsprechend Vorprodukt VP 1.2 wurden noch die folgenden Chloracethydroxamsäuren analog Beispiel 1 erhalten:

VP 26 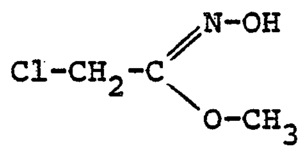 Fp. 62°C

VP 27 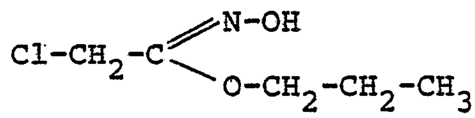 $n_D^{20} = 1,4663$

Verwendungsbeispiele

In den nachfolgenden Verwendungsbeispielen wurden die folgenden Wirkstoffe als Vergleichsverbindungen herangezogen:

(A) 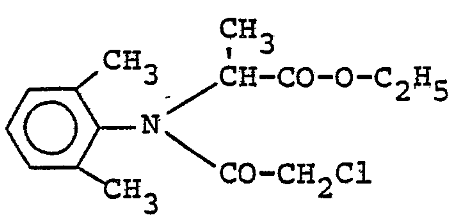

(B) 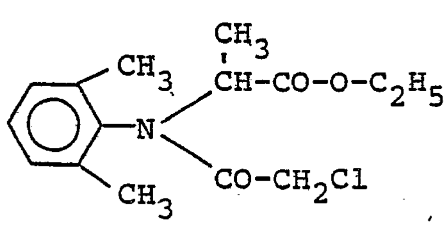

Beispiel A

Phytophthora-Test (Tomaten) / Protektiv

Lösungsmittel: 4,7 Gew.-Teile Aceton
Emulgator: 0,3 Gew.-Teile Alkyl-aryl-polyglykolether
Wasser: 95 Gew.-Teile

Man vermischt die für die gewünschte Wirkstoffkonzentration in der Spritzflüssigkeit nötige Wirkstoffmenge mit der angegebenen Menge des Lösungsmittels und verdünnt das Konzentrat mit der angegebenen Menge Wasser, welches die genannten Zusätze enthält.

Mit der Spritzflüssigkeit bespritzt man junge Tomatenpflanzen mit 2 bis 4 Laubblättern bis zur Tropfnässe. Die Pflanzen verbleiben 24 Stunden bei 20°C und einer relativen Luftfeuchtigkeit von 70 % im Gewächshaus. Anschließend werden die Tomatenpflanzen mit einer wäßrigen Sporensuspension von Phytophthora infestans inokuliert. Die Pflanzen werden in eine Feuchtkammer mit einer 100 %igen Luftfeuchtigkeit und einer Temperatur von 18 bis 20°C gebracht.

Nach 5 Tagen wird der Befall der Tomatenpflanzen bestimmt. Die erhaltenen Boniturwerte werden auf Prozent Befall umgerechnet. 0 % bedeutet keinen Befall, 100 % bedeutet, daß die Pflanzen vollständig befallen sind.

Eine deutliche Überlegenheit in der Wirksamkeit gegenüber dem Stand der Technik zeigen in diesem Test z.B. die Verbindungen gemäß folgender Herstellungsbeispiele: 3, 4, 13, 14, 5, 15, 24, 21, 20, 17, 9, 8, 35, 34, 32, 28.

Beispiel B

Phytophthora-Test (Tomaten) / Systemisch

Lösungsmittel: 4,7 Gew.-Teile Aceton
Dispergiermittel: 0,3 Gew.-Teile Alkyl-aryl-polyglykolether
Wasser: 95 Gew.-Teile

Man vermischt die für die gewünschte Wirkstoffkonzentration in der Gießflüssigkeit nötige Wirkstoffmenge mit der angegebenen Menge des Lösungsmittels und verdünnt das Konzentrat mit der angegebenen Menge Wasser, welches die genannten Zusätze enthält.

In Einheitserde angezogene Tomatenpflanzen mit 2 bis 4 Laubblättern werden mit 10 ccm der Gießflüssigkeit in der angegebenen Wirkstoffkonzentration, bezogen auf 100 ccm Erde, gegossen.

Die so behandelten Pflanzen werden nach der Behandlung mit einer wäßrigen Sporensuspension von Phytophthora infestans inokuliert. Die Pflanzen werden in eine Feuchtekammer mit einer Luftfeuchtigkeit von 100 % und einer Temperatur von 18 bis 20°C gebracht. Nach 5 Tagen wird der Befall der Tomatenpflanzen bestimmt. Die erhaltenen Boniturwerte werden auf Prozent Befall umgerechnet. 0 % bedeutet keinen Befall, 100 % bedeutet, daß die Pflanzen vollständig befallen sind.

Eine deutliche Überlegenheit in der Wirksamkeit gegenüber dem Stand der Technik zeigen in diesem Test z.B. die Verbindungen gemäß folgender Herstellungsbeispiele: 3, 4, 13, 14, 5, 15, 24, 2, 21, 17, 8, 28.

Patentansprüche

1. Hydroximsäureester der Formel

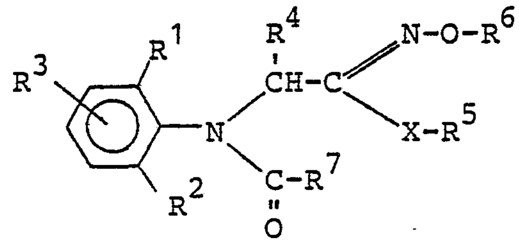

(I)

in welcher

$R^1$ für Wasserstoff, Alkyl und Alkoxy steht,

$R^2$ und $R^3$ für Wasserstoff, Alkyl, Alkoxy und Halogen stehen,

$R^4$ für Wasserstoff und Alkyl steht,

$R^5$ für Alkyl, Alkenyl, Alkinyl, Alkoxyalkyl und Halogenalkenyl steht,

$R^6$ für Wasserstoff, Alkyl, Alkenyl, Alkinyl, Alkoxyalkyl und Halogenalkenyl steht, und weiterhin noch

$R^4$ und $R^5$ sowie die Reste $R^5$ und $R^6$ gemeinsam unter Ringschluß für eine Alkylenbrücke stehen können, und

$R^7$ für Furyl, Tetrahydrofuryl, Thiophenyl und Tetrahydrothiophenyl, für gegebenenfalls durch Cyano oder Thiocyano substituiertes Alkyl, Alkenyl und Alkinyl steht, ferner für gegebenenfalls substituiertes Aryl, Cycloalkyl und Cycloalkenyl steht, für Halogenalkyl mit mehr als einem Halogenatom und schließlich für die Gruppierungen $-CH_2Az$, $-CH_2-OR^8$, $-CH_2-SR^8$, $-OR^8$, $-SR^8$, $-CH_2-OSO_2R^8$, $-COOR^8$, $CH_2-Ar$ und

$-CH_2-O-$ 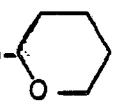 steht, wobei

$R^8$ für gegebenenfalls substituiertes Alkyl, Alkenyl, Alkinyl und Alkoxyalkyl steht,

Az für Pyrazol-1-yl, 1,2,4-Triazol-1-yl und Imidazol-1-yl steht,

Ar für gegebenenfalls substituiertes Phenyl steht, und

X für Sauerstoff oder Schwefel steht,

sowie für den Fall, daß $R^7$ für die Gruppe $-CH_2-Az$ steht, auch deren physiologisch verträglichen Säureadditions-Salze und Metallsalz-Komplexe.

2. Verbindungen der Formel I in Anspruch 1, in welcher

$R^1$, $R^2$ und $R^3$ für Wasserstoff, Methyl, Ethyl, Isopropyl, sek.-Butyl und tert.-Butyl, ferner für Methoxy, Ethoxy und Isopropoxy stehen, und darüber hinaus noch

$R^2$ und $R^3$ zusätzlich für Fluor, Chlor und Brom stehen können,

$R^4$ für Wasserstoff, Methyl und Ethyl steht,

$R^5$ und $R^6$ für Methyl, Ethyl, Propyl, Butyl, Isopropyl, Allyl, Methallyl, Monochlor-, Dichlor- und Trichlor-allyl, Propargyl, Methoxymethyl, Ethoxymethyl und Methoxyethyl stehen, und darüber hinaus noch

$R^6$ zusätzlich für Wasserstoff stehen kann, und schließlich

$R^4$ und $R^5$ oder $R^5$ und $R^6$ jeweils gemeinsam für eine Methylen-, Ethylen- oder Propylen-Brücke stehen können, und

$R^7$ für 2-Furyl, 2-Thienyl, 2-Tetrahydrofuryl, Methoxymethyl, Ethoxymethyl, Allyloxymethyl, Propargyloxymethyl, Methoxyethoxymethyl, Methylmercaptomethyl, Methoxy, Ethoxy, Methylmercapto, Methylsulfonyloxymethyl, Methoxycarbonyl, Ethoxycarbonyl, Methylvinyl, Dichlormethyl, Cyclopropyl, Cyclohexyl, Phenyl, Pyrazol-1-yl-methyl, Imidazol-1-yl-methyl, 1,2,4-Triazol-1-yl-methyl und Tetrahydropyran-2-yl-oxymethyl steht.

3. Verfahren zur Herstellung von Hydroximsäureestern der Formel

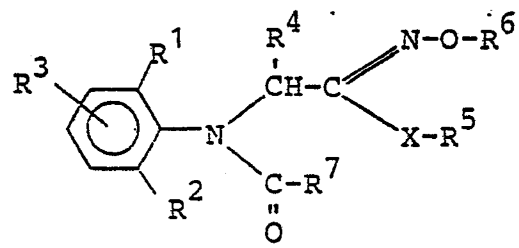

(I)

in welcher

$R^1$ für Wasserstoff, Alkyl und Alkoxy steht,

$R^2$ und $R^3$ für Wasserstoff, Alkyl, Alkoxy und Halogen stehen,

$R^4$ für Wasserstoff und Alkyl steht,

$R^5$ für Alkyl, Alkenyl, Alkinyl, Alkoxyalkyl und Halogenalkenyl steht,

$R^6$ für Wasserstoff, Alkyl, Alkenyl, Alkinyl, Alkoxyalkyl und Halogenalkenyl steht, und weiterhin noch

$R^4$ und $R^5$ sowie die Reste $R^5$ und $R^6$ gemeinsam unter Ringschluß für eine Alkylenbrücke stehen können, und

$R^7$ für Furyl, Tetrahydrofuryl, Thiophenyl und Tetrahydrothiophenyl, für gegebenenfalls durch Cyano oder Thiocyano substituiertes Alkyl, Alkenyl und Alkinyl steht, ferner für gegebenenfalls substituiertes Aryl, Cycloalkyl und Cycloalkenyl steht, für Halogenalkyl mit mehr als einem Halogenatom und schließlich für die Gruppierungen $-CH_2-Az$, $-CH_2-OR^8$, $-CH_2-SR^8$, $-OR^8$, $-SR^8$, $-CH_2-OSO_2R^8$, $-COOR^8$, $CH_2-Ar$ und

$-CH_2-O-$ 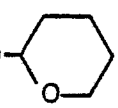 steht, wobei

$R^8$ für gegebenenfalls substituiertes Alkyl, Alkenyl, Alkinyl und Alkoxyalkyl steht,

Az für Pyrazol-1-yl, 1,2,4-Triazol-1-yl und Imidazol-1-yl steht,

Ar für gegebenenfalls substituiertes Phenyl steht, und

X für Sauerstoff oder Schwefel steht,

dadurch gekennzeichnet, daß man

a) Anilin-Derivate der Formel

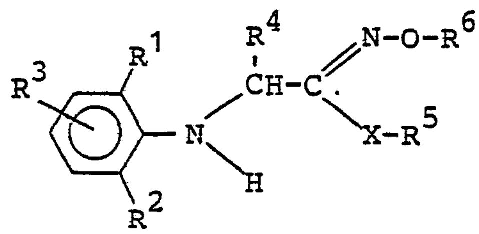

(II)

in welcher

$R^1, R^2, R^3, R^4, R^5, R^6$ und X die oben angegebene Bedeutung haben,

mit Säurechloriden oder -bromiden bzw. -anhydriden der Formeln

$$R^7-\underset{\overset{\|}{O}}{C}-Cl(Br) \qquad (IIIa)$$

bzw.

$$(R^7-\underset{\overset{\|}{O}}{C}-)_2O \qquad (IIIb)$$

in welchen

$R^7$ die oben angegebene Bedeutung hat,

in Gegenwart eines Verdünnungsmittels und gegebenenfalls in Gegenwart eines Säurebindemittels umsetzt, oder

b) Acylamino-hydroximsäureester der Formel

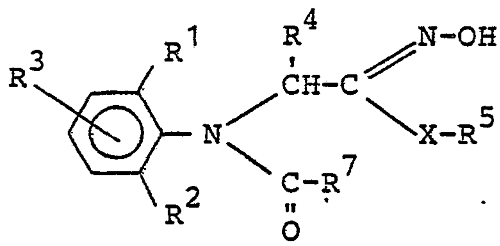

(IV)

in welcher

$R^1,R^2,R^3,R^4,R^5,R^7$ und X die oben angegebene Bedeutung haben,

gegebenenfalls in Form ihrer Metallsalze mit Alkylierungsmitteln der Formel

$$Y\text{-}R^6 \quad (V)$$

in welcher

$R^6$ die obengenannte Bedeutung, ausgenommen Wasserstoff, besitzt und

Y für Chlor, Brom oder die Gruppe $R^6-SO_2-O$ steht,

in Gegenwart eines organischen Verdünnungsmittels oder in Gegenwart eines organisch-wäßrigen Zweiphasensystems in Gegenwart eines Phasentransfer-Katalysators umsetzt, oder

c) Acylanilide der Formel

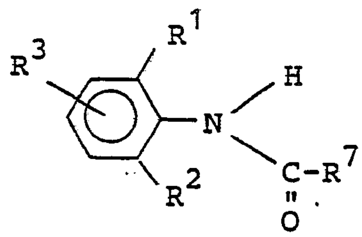

(VI)

in welcher

$R^1$, $R^2$, $R^3$ und $R^7$ die oben angegebene Bedeutung haben,

mit substituierten Alkylhydroximsäureestern der Formel

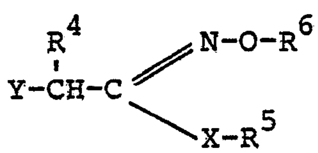

(VII)

in welcher

$R^4$, $R^5$, $R^6$, X und Y die oben angegebene Bedeutung haben,

in Gegenwart eines Säurebindemittels und gegebenenfalls in Gegenwart eines Verdünnungsmittels umsetzt, oder

d) für den Fall, daß $R^5$ und $R^6$ gemeinsam unter Ringschluß eine Alkylenbrücke bilden, Hydroxamsäuren der Formel

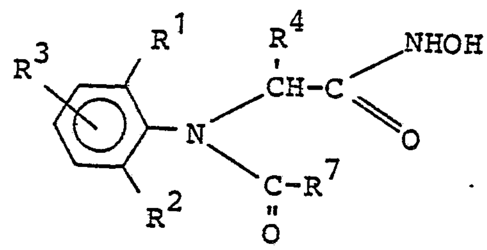

(VIII)

in welcher

$R^1$, $R^2$, $R^3$, $R^4$ und $R^7$ die oben angegebene Bedeutung haben,

in Gegenwart eines Verdünnungsmittels und in Gegenwart eines säurebindenden Mittels mit Verbindungen der Formel

$$Y-(CH_2)_n-Y \quad (IX)$$

in welcher

Y die obengenannte Bedeutung besitzt und

n eine ganze Zahl von 1 bis 4 darstellt,

umsetzt, oder

) Halogenacetanilide der Formel

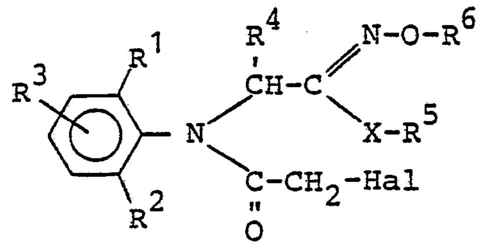

(X)

in welcher

$R^1, R^2, R^3, R^4, R^5, R^6$ und X die oben angegebene Bedeutung haben und

Hal für Chlor, Brom oder Jod steht,

it Verbindungen der Formel

$$B - R^9 \quad (XI)$$

n welcher

} für Wasserstoff oder ein Alkalimetall steht und

$R^9$ für Az und die Gruppierungen $-OR^8$ oder $-SR^8$ steht, wobei

Az und $R^8$ die obengenannte Bedeutung besitzen,

in Gegenwart eines Verdünnungsmittels und gegebenenfalls in Gegenwart eines Säurebinders umsetzt, und gegebenenfalls noch an solche nach den obigen Verfahrensvarianten a) bis e) erhaltenen Verbindungen der Formel I, bei denen $R^7$ für die Gruppe $CH_2$-Az steht, eine Säure oder ein Metallsalz addiert.

4. Fungizide Mittel, gekennzeichnet durch einen Gehalt an mindestens einem Hydroximsäureester der Formel I in Ansprüchen 1 und 3.

5. Verfahren zur Bekämpfung von Pilzen, dadurch gekennzeichnet, daß man Hydroximsäureester der Formel I in Ansprüchen 1 und 3 auf Pilze oder ihren Lebensraum einwirken läßt.

6. Verwendung von Hydroximsäureestern der Formel I in Ansprüchen 1 und 3 zur Bekämpfung von Pilzen.

7. Verfahren zur Herstellung von fungiziden Mitteln, dadurch gekennzeichnet, daß man Hydroximsäureester der Formel I in Ansprüchen 1 und 3 mit Streckmitteln und/oder oberflächenaktiven Mitteln vermischt.